# EUROPEAN PATENT SPECIFICATION

(11) **EP 3 096 774 B1**
(45) Date of publication and mention of the grant of the patent: **24.03.2021**
(21) Application number: 15740849.3
(22) Date of filing: 21.01.2015
(51) Int. Cl.: C07K 14/74, A61K 38/17, A61K 39/385, C07K 19/00, G01N 33/569, C12Q 1/68

(54) **CELLULAR PLATFORM FOR RAPID AND COMPREHENSIVE T-CELL IMMUNOMONITORING**
ZELLULARE PLATTFORM ZUR SCHNELLEN UND UMFASSENDEN T-ZELLEN-IMMUNÜBERWACHUNG
PLATE-FORME CELLULAIRE D'IMMUNOSURVEILLANCE RAPIDE ET COMPLÈTE DES LYMPHOCYTES T

(30) Priority: 21.01.2014 US 201461929651 P
(43) Date of publication of application: 30.11.2016
(73) Proprietor: Albert Einstein College of Medicine, Bronx, NY 10461 (US)
(72) Inventor: SEIDEL, Ronald D. III, Nattick, Massachusetts, 01760 (US); CHAPARRO, Rodolfo, Bronx, NY 10461 (US); HILLERICH, Brandan S., Arlington, Massachusetss 02474 (US); ALMO, Steven C., Pelham, NY 10803 (US)
(74) Representative: Mewburn Ellis LLP
(86) International application number: PCT/US2015/012160
(87) International publication number: WO 2015/112541

(56) References cited:
- WO-A2-2007/136778
- WO-A2-2008/019888
- WO-A2-2012/127464
- US-A1- 2004 161 817
- US-A1- 2008 219 947
- US-A1- 2011 268 737
- US-A1- 2012 121 577
- GAL CAFRI ET AL: "Development of novel genetic cancer vaccines based on membrane-attached [beta] 2 microglobulin : Cafri et al.", ANNALS OF THE NEW YORK ACADEMY OF SCIENCES, vol. 1283, no. 1, 6 April 2013 (2013-04-06), pages 87-90, XP055394144, US ISSN: 0077-8923, DOI: 10.1111/nyas.12017
- A. MARGALIT ET AL: "Induction of Antitumor Immunity by CTL Epitopes Genetically Linked to Membrane-Anchored 2-Microglobulin", THE JOURNAL OF IMMUNOLOGY, vol. 176, no. 1, 19 December 2005 (2005-12-19), pages 217-224, XP55394142, US ISSN: 0022-1767, DOI: 10.4049/jimmunol.176.1.217
- SOJUNG KIM ET AL: "Single chain MHC I trimer-based DNA vaccines for protection againstinfection", VACCINE, ELSEVIER, AMSTERDAM, NL, vol. 30, no. 12, 4 January 2012 (2012-01-04), pages 2178-2186, XP028460662, ISSN: 0264-410X, DOI: 10.1016/J.VACCINE.2012.01.012 [retrieved on 2012-01-19]
- HANSEN N J V: "PHAGE DISPLAY OF PEPTIDE/MAJOR HISTOCOMPATIBILITY CLASS I COMPLEXES", EUROPEAN JOURNAL OF IMMUNOLOGY,, vol. 31, 1 January 2001 (2001-01-01), pages 32-38, XP001028492, ISSN: 0014-2980, DOI: 10.1002/1521-4141(200101)31:1<32::AID-IMMU 32>3.0.CO;2-6
- Donatien Kamdem Toukam ET AL: "Targeting Antibody Responses to the Membrane Proximal External Region of the Envelope Glycoprotein of Human Immunodeficiency Virus", PLoS ONE, vol. 7, no. 5, 30 May 2012 (2012-05-30), page e38068, XP055337233, DOI: 10.1371/journal.pone.0038068
- WEN FEI ET AL: "Construction and Screening of an Antigen-Derived Peptide Library Displayed on Yeast Cell Surface for CD4(+) T Cell Epitope Identification", 2013, IMMUNOPROTEOMICS: METHODS AND PROTOCOLS HUMANA PRESS INC, 999 RIVERVIEW DR, STE 208, TOTOWA, NJ 07512-1165 USA SERIES : METHODS IN MOLECULAR BIOLOGY (ISSN 1064-3745(PRINT)), PAGE(S) 245-264, XP009506613, ISSN: null
- Ran Taube ET AL: "Lentivirus Display: Stable Expression of Human Antibodies on the Surface of Human Cells and Virus Particles", PLoS ONE, vol. 3, no. 9, 11 September 2008 (2008-09-11), page e3181, XP055027886, DOI: 10.1371/journal.pone.0003181
- Y. Wang ET AL: "Using a baculovirus display library to identify MHC class I mimotopes", PROCEEDINGS OF THE NATIONAL ACADEMY OF SCIENCES OF THE UNITED STATES OF AMERICA, vol. 102, no. 7, 15 February 2005 (2005-02-15), pages 2476-2481, XP55590862, US ISSN: 0027-8424, DOI: 10.1073/pnas.0409798102
- Frances Crawford ET AL: "Use of baculovirus MHC/peptide display libraries to characterize T-cell receptor ligands", Immunological Reviews, vol. 210, no. 1, 1 April 2006 (2006-04-01) , pages 156-170, XP055217192, ISSN: 0105-2896, DOI: 10.1111/j.0105-2896.2006.00365.x

## Description

### BACKGROUND OF THE INVENTION

Throughout this application various publications are referred to in square brackets. Full citations for these references may be found at the end of the specification.

Remarkable growth has been made over the past decade in the development and application of genomic [13-15] and proteomic technologies [16-19] for the identification of molecular signatures associated with clinically important disease states and differential responses to therapies. These advances hold the promise of personalized diagnostics [20]. As an example, Adaptive Biotechnologies utilizes high throughput sequencing of the T cell receptor (TCR) beta chain hypervariable region to provide researchers with a full analysis of the TCR repertoire within a sample [21]. This venture capital-funded effort is presently a fee-for-service enterprise, with a projected market depth for biomarker discovery of $300 million. The rapid progress in high throughput technologies has been paralleled by the stepwise clinical development of biologics (e.g., monoclonal antibodies, therapeutic proteins, and peptides) [22-25], and has revolutionized the treatment of immune borne diseases. For example, unlike traditional vaccines, which boost immunity primarily via antibody responses, Genocea Biosciences is developing novel, biologics-based vaccines focused on generating robust T-cell responses against intracellular pathogens. Likewise, Apitope, a European biotechnology company, is developing therapeutic peptides for the treatment of autoimmune diseases in which T-cells play a key pathogenic role. Aptiope recently partnered with Merck-Serono for the continued development of their flagship MS peptide therapeutic. Efforts seeking to monitor, enhance or alter T-cell immunity will depend heavily on the ability to identify clinically relevant T-cell epitopes.

At the core of the molecular events comprising a CD8-mediated adaptive immune response is the engagement of the T-cell receptor (TCR) with a small peptide antigen non-covalently presented by a major histocompatibility complex (MHC) molecule, referred to as a T-cell epitope. This represents the immune system's targeting mechanism and is a requisite molecular interaction for T-cell activation and effector function. During T-cell development, a genomic editing process results in the expression of a unique TCR on every immune cell, with an estimated depth of over 3 million unique sequences [1] and accounts for the enormous diversity of antigens to which T-cells can respond. However, T-cell epitopes have historically been difficult to study, as each TCR requires individual characterization with respect to specificity as well as the development of custom reagents (e.g., tetramers) for further study. Clinically, this challenge is compounded by the fact that immune responses typically involve many T-cell specificities, for example targeting multiple viral antigens to effect viral clearance for a single pathogen response. Thus, the ability to systematically identify the entire ensemble of epitopes for a given disease state represents a unique opportunity for the development of diagnostics and potential highly targeted therapeutics against infectious diseases, autoimmunity and cancers.

There exists a number of experimental approaches for epitope discovery, which include the screening of expression [3, 4] and synthetic peptide libraries [5, 6], positional scanning libraries [7], pMHC microarrays [8], as well as mass spectrometric identification of naturally-occurring epitopes [9-11]. Marrack and Kappler developed a baculovirus-infected insect cell strategy as a display platform for class I MHC molecules covalently bound to a library of potential peptide mimotopes [4]. Mimotopes differ in sequence from the unknown peptide epitope, but they are nevertheless recognized by the specific CD8 T-cell receptor. However, it is often challenging to link the identified mimotope to the natural epitope. Moreover, the baculoviral display system requires 5-10 time-consuming rounds of cell sorting, viral generation, expansion and reinfection to resolve a mimotope, coupled with a requirement to purify and tetramerize the cognate TCR. Partially addressing these issues, Newell et al leveraged heavy-isotope tagging of traditional MHC tetramers combined with flow cytometry and mass spectroscopy (termed mass cytometry) to screen a small set of pMHC tetramer combinations directly from a human blood sample with astonishing sensitivity, although this technology is presently limited to ∼100 such combinations per assay [12]. Each of these approaches has contributed valuable insights into T-cell epitopes; however, these methods are slow, labor-intensive and require a high degree of user skill.

WO2007/136778 discloses fusion proteins comprising a cytomegalovirus human MHC-restricted peptide, a human β-2 microglobulin, a HLA-A2 chain of a human MHC class I molecule, a variable region from a heavy chain of a scFv fragment of an antibody and a variable region from a light chain of such scFv fragment; wherein the antibody from which the scFv fragment is derived specifically binds to mesothelin.

US2008/0219947 discloses a single chain trimer which comprises an MHC antigen peptide sequence, a β-2 microglobulin sequence and a full length MHC class I heavy chain. They further disclose methods of using these trimers to expand antigen-specfic T cell populations.

Wang, Y. et al. (2005) PNAS, vol. 102, no. 7, 2476 - 2481 and Crawford, F. et al. (2006) Immunological Reviews, vol. 210, no. 1, 156 - 170 disclosed a baculovirus-based display system for identifying antigen mimotopes for MHC class I-specific T cells. The mouse MHC class I molecule, D^{d}, was displayed on baculovirus-infected insect cells with a library of 9- and 10-mer peptides tethered via a flexible linker to the N terminus of 132 microglobulin. As a test case, the library was screened by flow cytometry by using a multimeric fluorescent apTCR from a mouse T cell specific for D^{d}plus an unknown self peptide.

The present invention addresses this need for new and improved technologies for the efficient and systematic identification of the repertoire of T-cell epitopes.

### SUMMARY OF THE INVENTION

This invention provides a (i) lentivirus-like particle or (ii) lentivirus, which lentivirus-like particle or a lentivirus is physically associated with an expression product via a mammalian cell membrane portion associated with the lentivirus-like particle or lentivirus and the mammalian transmembrane domain of the expression product, the expression product comprising in N-terminal to C-terminal order:
a 5 to 20 amino acid peptide, contiguous with
a first linker peptide sequence, contiguous with
a beta 2 microglobulin sequence, contiguous with
a second linker peptide sequence, contiguous with
a class I Major Histocompatibility Complex (MHC) heavy chain sequence, contiguous with
a third linker peptide sequence, contiguous with
a fluorescent protein or a sequence of an immunoglobulin Fc domain, contiguous with
a fourth linker peptide sequence, contiguous with
the mammalian transmembrane domain, contiguous with
a lentiviral packaging sequence comprising the GP41 envelop protein residues 706-713.

This invention also provides an isolated suspension-adapted mammalian cell expressing an expression product of a heterologous nucleic acid transduced or transfected therein, or a membrane-bound portion of such cell expressing the expression product, which expression product comprises, in N-terminal to C-terminal order:
a 5 to 20 amino acid peptide, contiguous with
a first linker peptide sequence, contiguous with
a beta 2 microglobulin sequence, contiguous with
a second linker peptide sequence, contiguous with
a class I Major Histocompatibility Complex heavy chain sequence, contiguous with
a third linker peptide sequence, contiguous with
a fluorescent protein or a sequence of an immunoglobulin Fc domain, contiguous with
a fourth linker peptide sequence, contiguous with
a mammalian transmembrane domain, contiguous with
a lentiviral packaging sequence comprising the GP41 envelop protein residues 706-713.

Described herein is a recombinant nucleic acid comprising, in 5' to 3' order:
a sequence encoding a leader oligonucleotide sequence, contiguous with
an oligonucleotide sequence encoding an 8, 9, 10, 11 or 12 amino acid peptide, contiguous with
an oligonucleotide sequence encoding a first linker, contiguous with
an oligonucleotide sequence encoding a beta 2 microglobulin sequence, contiguous with
an oligonucleotide sequence encoding a second linker, contiguous with
a Major Histocompatibility Complex heavy chain sequence, contiguous with
a third linker peptide sequence, contiguous with
a fluorescent protein, contiguous with
a fourth linker peptide sequence, contiguous with
a Major Histocompatibility Complex heavy chain transmembrane domain.

Also provided is a method of identifying a T-cell epitope comprising:
contacting a T-cell with a plurality, comprising at least two cells, of isolated suspension-adapted cells according to any one of claims 5-12, or a membrane-bound portion of such cells expressing the expression product, or with lentivirus-like particles or lentiviruses according to any one of claims 1-3 associated with a membrane portion of such cells;
recovering T-cell(s) which have formed a conjugate with a suspension-adapted cell or membrane-bound portions;
recovering DNA from the suspension-adapted cell(s);
sequencing the recovered DNA; and
identifying the 5 to 20 amino acid peptide(s) encoded for in the DNA, so as to thereby identify a T-cell epitope.

Also described herein is an isolated suspension-adapted cell transduced by or transfected with a heterologous nucleic acid comprising, in 5' to 3' order:
a leader oligonucleotide sequence, contiguous with
an oligonucleotide sequence encoding an 8, 9, 10, 11 or 12 amino acid peptide, contiguous with
an oligonucleotide sequence encoding a first linker, contiguous with
an oligonucleotide sequence encoding a beta 2 microglobulin sequence, contiguous with
an oligonucleotide sequence encoding a second linker, contiguous with
an oligonucleotide sequence encoding a Major Histocompatibility Complex heavy chain sequence, contiguous with
an oligonucleotide sequence encoding a third linker, contiguous with
an oligonucleotide sequence encoding a Major Histocompatibility Complex heavy chain transmembrane domain.

Also provided is an isolated suspension-adapted cell expressing an expression product of a heterologous nucleic acid transduced or transfected therein, which expression product comprises, in N-terminal to C-terminal order:
an 8, 9, 10, 11 or 12 amino acid peptide, contiguous with
a first linker peptide sequence, contiguous with
a beta 2 microglobulin sequence, contiguous with
a second linker peptide sequence, contiguous with
a Major Histocompatibility Complex heavy chain sequence, contiguous with
a third linker peptide sequence, contiguous with
a Major Histocompatibility Complex heavy chain transmembrane domain.

A recombinant nucleic acid is provided comprising, in 5' to 3' order:
a sequence encoding a leader oligonucleotide sequence, contiguous with
an oligonucleotide sequence encoding an 8, 9, 10, 11 or 12 amino acid peptide, contiguous with
an oligonucleotide sequence encoding a first linker, contiguous with
an oligonucleotide sequence encoding a beta 2 microglobulin sequence, contiguous with
an oligonucleotide sequence encoding a second linker, contiguous with
a Major Histocompatibility Complex heavy chain sequence, contiguous with
a third linker peptide sequence, contiguous with
a Major Histocompatibility Complex heavy chain transmembrane domain.

Also described is a method of identifying a T-cell epitope comprising contacting a T-cell with a plurality of isolated suspension-adapted cells comprising at least two cells, each expressing an expression product of a heterologous nucleic acid transduced or transfected therein, each of which expression products comprises an 8, 9, 10, 11 or 12 amino acid peptide, contiguous with first linker peptide sequence, contiguous with a beta 2 microglobulin sequence, contiguous with a second linker peptide sequence, contiguous with a Major Histocompatibility Complex heavy chain sequence, contiguous with a third linker peptide sequence, contiguous with a Major Histocompatibility Complex heavy chain transmembrane domain, wherein the plurality of isolated suspension-adapted cells expresses at least two different encoded 8, 9, 10, 11 or 12 amino acid peptides among the cells thereof under conditions permitting T-cells to conjugate with the 8, 9, 10, 11 or 12 amino acid peptides;
recovering T-cell(s) which have formed a conjugate with a suspension-adapted cell;
recovering DNA from the recovered T-cell(s);
sequencing the recovered DNA;
identifying the 8, 9, 10, 11 or 12 amino acid peptide(s) encoded for in the DNA,
so as to thereby identify a T-cell epitope.

Also described is an isolated suspension-adapted cell transduced by or transfected with a heterologous nucleic acid comprising, in 5' to 3' order:
a leader oligonucleotide sequence, contiguous with
an oligonucleotide sequence encoding a 5 to 20 amino acid peptide, contiguous with
an oligonucleotide sequence encoding a first linker, contiguous with
an oligonucleotide sequence encoding a beta 2 microglobulin sequence, contiguous with
an oligonucleotide sequence encoding a second linker, contiguous with
an oligonucleotide sequence encoding a Major Histocompatibility Complex heavy chain sequence, contiguous with
an oligonucleotide sequence encoding a third linker, contiguous with
an oligonucleotide sequence encoding a fluorescent protein or encoding an immunoglobulin Fc domain, contiguous with
an oligonucleotide sequence encoding a fourth linker, contiguous with
an oligonucleotide sequence encoding a mammalian transmembrane domain.

Also described is an isolated suspension-adapted cell expressing an expression product of a heterologous nucleic acid transduced or transfected therein, or a membrane-bound portion of such cell expressing the expression product, which expression product comprises, in N-terminal to C-terminal order:
a 5 to 20 amino acid peptide, contiguous with
a first linker peptide sequence, contiguous with
a beta 2 microglobulin sequence, contiguous with
a second linker peptide sequence, contiguous with
a Major Histocompatibility Complex heavy chain sequence, contiguous with
a third linker peptide sequence, contiguous with
a fluorescent protein or a sequence of an immunoglobulin Fc domain, contiguous with
a fourth linker peptide sequence, contiguous with
a mammalian transmembrane domain.

Also described is an isolated suspension-adapted cell expressing an expression product of a heterologous nucleic acid transduced or transfected therein, or a membrane-bound portion of such cell expressing the expression product, which expression product comprises, in N-terminal to C-terminal order:
a 5 to 20 amino acid peptide, contiguous with
a first linker peptide sequence, contiguous with
a beta 2 microglobulin sequence, contiguous with
a second linker peptide sequence, contiguous with
a Major Histocompatibility Complex heavy chain sequence, contiguous with
a third linker peptide sequence, contiguous with
a fluorescent protein or a sequence of an immunoglobulin Fc domain, contiguous with
a fourth linker peptide sequence, contiguous with
a mammalian transmembrane domain.

A plurality of the isolated suspension-adapted cells or a plurality of membrane-bound portions of such cells expressing the expression product, wherein the plurality comprises at least two different encoded 5 to 20 amino acid peptides, is also described herein.

A (i) virus-like particle or (ii) virus, produced by an isolated suspension-adapted cell as described herein is disclosed, which virus like particle or a virus is physically associated via a cell membrane portion having attached thereto, by a mammalian transmembrane domain, an expression product comprising in N-terminal to C-terminal order:
a 5 to 20 amino acid peptide, contiguous with
a first linker peptide sequence, contiguous with
a beta 2 microglobulin sequence, contiguous with
a second linker peptide sequence, contiguous with
a Major Histocompatibility Complex heavy chain sequence, contiguous with
a third linker peptide sequence, contiguous with
a fluorescent protein or a sequence of an immunoglobulin Fc domain, contiguous with
a fourth linker peptide sequence, contiguous with
the mammalian transmembrane domain, contiguous with
a lentiviral packaging sequence as defined in the claims.

A plurality of the virus-like particles described, or of the viruses described, is also disclosed.

Also described is a recombinant nucleic acid comprising, in 5' to 3' order:
a sequence encoding a leader oligonucleotide sequence, contiguous with
an oligonucleotide sequence encoding a 5 to 20 amino acid peptide, contiguous with
an oligonucleotide sequence encoding a first linker, contiguous with
an oligonucleotide sequence encoding a beta 2 microglobulin sequence, contiguous with
an oligonucleotide sequence encoding a second linker, contiguous with
an oligonucleotide sequence encoding a Major Histocompatibility Complex heavy chain sequence, contiguous with
an oligonucleotide sequence encoding a third linker peptide sequence, contiguous with
an oligonucleotide sequence encoding a fluorescent protein or an immunoglobulin Fc domain, contiguous with
an oligonucleotide sequence encoding a fourth linker peptide sequence, contiguous with an oligonucleotide sequence encoding a mammalian transmembrane domain.

Also described is an isolated suspension-adapted cell transduced by or transfected with a heterologous nucleic acid comprising, in 5' to 3' order:
an oligonucleotide sequence encoding a first B2M leader sequence,
contiguous with an oligonucleotide sequence encoding a preselected 5 to 20 amino acid peptide, contiguous with an oligonucleotide sequence encoding a first amino acid linker sequence, contiguous with an oligonucleotide sequence encoding a sequence of amino acids identical to a human native B2M peptide sequence,
contiguous with an oligonucleotide sequence encoding a second amino acid linker sequence,
contiguous with an oligonucleotide sequence encoding a preselected second peptide sequence,
contiguous with an oligonucleotide sequence encoding a third amino acid linker,
contiguous with an oligonucleotide sequence encoding a second B2M leader sequence,
contiguous with an oligonucleotide sequence encoding a sequence of amino acids identical to a MHC heavy chain,
contiguous with an oligonucleotide sequence encoding a fourth amino acid linker,
contiguous with an oligonucleotide sequence encoding a sequence of amino acids identical to an immunoglobulin Fc domain,
contiguous with an oligonucleotide sequence encoding a fifth linker,
contiguous with an oligonucleotide sequence encoding a mammalian transmembrane domain.

Also described is an isolated suspension-adapted cell expressing an expression product of a heterologous nucleic acid transduced or transfected therein, or a membrane-bound portion of such cell expressing the expression product, which expression product comprises a recombinant polypeptide construct comprising (i) a preselected 5 to 20 amino acid peptide bound by a first amino acid linker sequence contiguous with a sequence of amino acids comprising a sequence identical to a human native B2M peptide sequence contiguous with a second amino acid linker sequence contiguous with a preselected second peptide sequence, wherein (i) is bound by one, or more than one, disulfide bond to (ii) a sequence of amino acids having the sequence of a MHC heavy chain contiguous with a fourth amino acid linker sequence contiguous with a sequence of amino acids identical to an immunoglobulin Fc domain contiguous with a fifth amino acid linker, contiguous with a mammalian transmembrane domain.

Also described is an isolated suspension-adapted cell transduced by or transfected with a virus, plasmid or viral vector comprising a heterologous nucleic acid comprising, in 5' to 3' order:
an oligonucleotide sequence encoding a first B2M leader sequence,
contiguous with an oligonucleotide sequence encoding a preselected 5 to 20 amino acid peptide, contiguous with an oligonucleotide sequence encoding a first amino acid linker sequence, contiguous with an oligonucleotide sequence encoding a sequence of amino acids identical to a human native B2M peptide sequence,
contiguous with an oligonucleotide sequence encoding a second amino acid linker sequence,
contiguous with an oligonucleotide sequence encoding a preselected second peptide sequence,
contiguous with an oligonucleotide sequence encoding a third amino acid linker,
contiguous with an oligonucleotide sequence encoding a second B2M leader sequence,
contiguous with an oligonucleotide sequence encoding a sequence of amino acids identical to a MHC heavy chain,
contiguous with an oligonucleotide sequence encoding a fourth amino acid linker,
contiguous with an oligonucleotide sequence encoding a sequence of amino acids identical to an immunoglobulin Fc domain,
contiguous with an oligonucleotide sequence encoding a fifth linker,
contiguous with an oligonucleotide sequence encoding a mammalian transmembrane domain, contiguous with an oligonucleotide encoding a viral packaging sequence.

Further disclosed is a (i) virus like particle or (ii) virus, produced by cell described herein, which virus like particle or a virus is physically associated via a cell membrane portion having attached thereto, by a mammalian transmembrane domain, an expression product comprising in N-terminal to C-terminal order:
a recombinant polypeptide construct comprising (i) a preselected 5 to 20 amino acid peptide bound by a first amino acid linker sequence contiguous with a sequence of amino acids comprising a sequence identical to a human native B2M peptide sequence contiguous with a second amino acid linker sequence contiguous with a preselected second peptide sequence,
wherein (i) is bound by one, or more than one, disulfide bond to (ii) a sequence of amino acids having the sequence of a MHC heavy chain contiguous with a fourth amino acid linker sequence contiguous with a sequence of amino acids identical to an immunoglobulin Fc domain contiguous with a fifth amino acid linker, contiguous with a mammalian transmembrane domain, contiguous with a viral packaging sequence.

Also described is a recombinant nucleic acid comprising, in 5' to 3' order:
an oligonucleotide sequence encoding a first B2M leader sequence,
contiguous with an oligonucleotide sequence encoding a preselected 5 to 20 amino acid peptide, contiguous with an oligonucleotide sequence encoding a first amino acid linker sequence, contiguous with an oligonucleotide sequence encoding a sequence of amino acids identical to a human native B2M peptide sequence,
contiguous with an oligonucleotide sequence encoding a second amino acid linker sequence,
contiguous with an oligonucleotide sequence encoding a preselected second peptide sequence,
contiguous with an oligonucleotide sequence encoding a third amino acid linker,
contiguous with an oligonucleotide sequence encoding a second B2M leader sequence,
contiguous with an oligonucleotide sequence encoding a sequence of amino acids identical to a MHC heavy chain,
contiguous with an oligonucleotide sequence encoding a fourth amino acid linker,
contiguous with an oligonucleotide sequence encoding a sequence of amino acids identical to an immunoglobulin Fc domain,
contiguous with an oligonucleotide sequence encoding a fifth linker,
contiguous with an oligonucleotide sequence encoding a mammalian transmembrane domain.

Further disclosed herein is a method of identifying a T-cell epitope comprising contacting a T-cell with a plurality of isolated suspension-adapted cells comprising at least two cells, or a membrane-bound portion of such cells expressing the expression product, each cell or membrane bound portion expressing an expression product of a heterologous nucleic acid transduced or transfected therein, each of which expression products comprises a 5 to 20 amino acid peptide, contiguous with first linker peptide sequence, contiguous with a beta 2 microglobulin sequence, contiguous with a second linker peptide sequence, contiguous with a Major Histocompatibility Complex heavy chain sequence, contiguous with a third linker peptide sequence, contiguous with a fluorescent protein or an immunoglobulin Fc domain, contiguous with a fourth linker peptide sequence, contiguous with a mammalian transmembrane domain, wherein the plurality of isolated suspension-adapted cells or membrane-bound portions expresses at least two different encoded 5 to 20 amino acid peptides among the cells or membrane-bound portions under conditions permitting T-cells to conjugate with the 5 to 20 amino acid peptides;
recovering T-cell(s) which have formed a conjugate with a suspension-adapted cell or membrane-bound portions;
recovering DNA from the suspension-adapted cell(s);
sequencing the recovered DNA;
identifying the 5 to 20 amino acid peptide(s) encoded for in the DNA,
so as to thereby identify a T-cell epitope.

Also described herein is a method of identifying a T-cell epitope comprising contacting a T-cell with a plurality of isolated suspension-adapted cells comprising at least two cells, or membrane-bound portions thereof, each expressing an expression product of a heterologous nucleic acid transduced or transfected therein, each of which expression products comprises (i) a preselected 5 to 20 amino acid peptide bound by a first amino acid linker sequence contiguous with a sequence of amino acids comprising a sequence identical to a human native B2M peptide sequence contiguous with a second amino acid linker sequence contiguous with a preselected second peptide sequence,
wherein (i) is bound by one, or more than one, disulfide bond to (ii) a sequence of amino acids having the sequence of a MHC heavy chain contiguous with a fourth amino acid linker sequence contiguous with a sequence of amino acids identical to an immunoglobulin Fc domain contiguous with a fifth amino acid linker, contiguous with a mammalian transmembrane domain, wherein the plurality of isolated suspension-adapted cells or membrane-bound portions thereof expresses at least two different encoded 5 to 20 amino acid peptides among the cells or portions under conditions permitting T-cells to conjugate with the 5 to 20 amino acid peptides;
recovering T-cell(s) which have formed a conjugate with a suspension-adapted cell or membrane-bound portion;
recovering DNA from the suspension-adapted cell(s);
sequencing the recovered DNA;
identifying the 5 to 20 amino acid peptide(s) encoded for in the DNA,
so as to thereby identify a T-cell epitope.

A method of identifying a T-cell epitope is also described herein, the method comprising
contacting a T-cell with a plurality of isolated suspension-adapted cells comprising at least two cells, or virus-like particle or viruses associated with a membrane portion of such cells, the cells or membrane bound portion expressing an expression product of a heterologous nucleic acid transduced or transfected therein, each of which expression products comprises a 5 to 20 amino acid peptide, contiguous with first linker peptide sequence, contiguous with a beta 2 microglobulin sequence, contiguous with a second linker peptide sequence, contiguous with a Major Histocompatibility Complex heavy chain sequence, contiguous with a third linker peptide sequence, contiguous with a fluorescent protein or an immunoglobulin Fc domain, contiguous with a fourth linker peptide sequence, contiguous with a mammalian transmembrane domain, contiguous with a viral packaging sequence, wherein the plurality of isolated suspension-adapted cells or of virus-like particles or viruses associated with the membrane portion of the cells, expresses at least two different encoded 5 to 20 amino acid peptides among the cells or virus-like particles or viruses under conditions permitting T-cells to conjugate with the 5 to 20 amino acid peptides;
recovering T-cell(s) which have formed a conjugate with a suspension-adapted cell, virus-like particle or virus of the plurality;
recovering DNA from the suspension-adapted cell or RNA from the virus-like particle or virus;
sequencing the recovered DNA or RNA;
identifying the 5 to 20 amino acid peptide(s) encoded for in the DNA or RNA,
so as to thereby identify a T-cell epitope.

A further method of identifying a T-cell epitope is described. The method comprising
contacting a T-cell with a plurality of isolated suspension-adapted cells comprising at least two cells, or virus-like particles or viruses associated with a membrane portion of such a cells, each expressing an expression product of a heterologous nucleic acid transduced or transfected therein, each of which expression products comprises (i) a preselected 5 to 20 amino acid peptide bound by a first amino acid linker sequence contiguous with a sequence of amino acids comprising a sequence identical to a human native B2M peptide sequence contiguous with a second amino acid linker sequence contiguous with a preselected second peptide sequence,
wherein (i) is bound by one, or more than one, disulfide bond to (ii) a sequence of amino acids having the sequence of a MHC heavy chain contiguous with a fourth amino acid linker sequence contiguous with a sequence of amino acids identical to an immunoglobulin Fc domain contiguous with a fifth amino acid linker, contiguous with a mammalian transmembrane domain, wherein the plurality of isolated suspension-adapted cells, virus-like particles or viruses expresses at least two different encoded 5 to 20 amino acid peptides among the cells thereof under conditions permitting T-cells to conjugate with the 5 to 20 amino acid peptides;
recovering T-cell(s) which have formed a conjugate with a suspension-adapted cell, virus-like particle or virus associated membrane portion;
recovering DNA from the suspension-adapted cell or RNA from the virus-like particle or virus;
sequencing the recovered DNA or RNA;
identifying the 5 to 20 amino acid peptide(s) encoded for in the DNA or RNA,
so as to thereby identify a T-cell epitope.

### BRIEF DESCRIPTION OF THE DRAWINGS

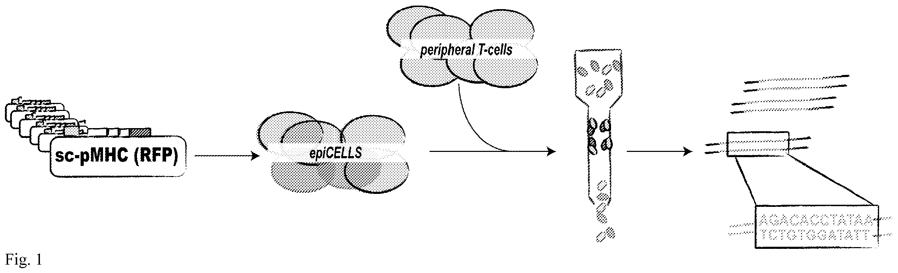

Figure 1. General overview of the *"epiCELL"* immunomonitoring platform for high-throughput identification of CD8⁺ T-cell epitopes. A library of sc-pMHC vectors is pooled and transfected *en masse* into suspension adapted HEK293 cells, generating the *epiCELL* pool. The pooled expression library is mixed with patient derived peripheral T-cells and allowed to form conjugates, which are recovered by magnetic separation or more traditional flow cytometric sorting procedures. Magnetic beads, if used, can be from any commercial source, including Dynabeads® CD8 from Life technologies and CD8 microbeads (MACS) from Miltenyi. For example, superparamagnetic beads coupled with an anti-human CD8 antibody that enable easy isolation or depletion of human CD8+ T cells directly from any sample, including whole blood, bone marrow, buffy coat, mononuclear cells (MNC), and tissue digests. The epitope sequences from the enriched pool members are amplified by PCR using universal primers and subjected to next-generation deep sequencing to identify epitopes enriched by the capture process.

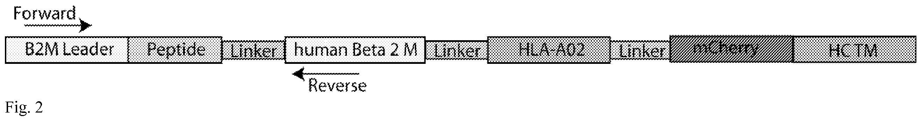

Figure 2. Design of membrane-anchored class I sc-pMHC construct. Construct utilizes a native human B2M leader sequence immediately followed by a candidate epitope (labeled as peptide), further coupled to the native B2M molecule, the human HLA-A02:01, and a surface exposed mCherry expression proxy through linker regions (4 repeats of GGGGS for each of the first second and third linkers and optionally a 2 repeat GGGGS for as a fourth linker between the fluorescent protein and HC TM). The entire construct is held in the membrane through a native Class-I Heavy Chain transmembrane domain (HC TM). Universal primers (labeled Forward, Reverse) are used to amplify the unique 27-nucleotide sequence (9-mer peptide) following T-cell challenge to directly identify disease relevant epitopes.

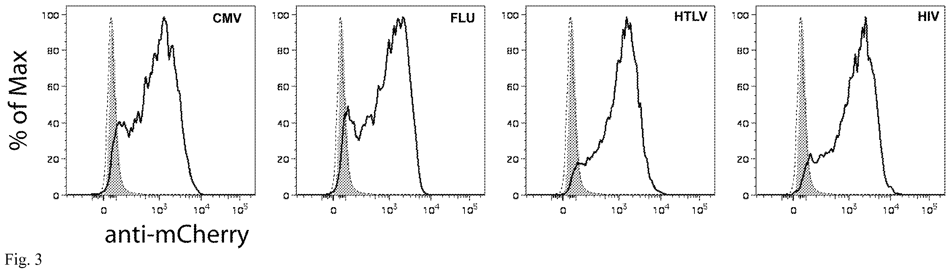

Figure 3. Surface expression validation of MHC controls. Expression validation of 4 known pathogenic HLA-A02 restricted epitopes linked to 4 independent viral pathogens displayed in our sc-pMHC *epiCELL* platform. The constructs being examined are CMV pp65 protein residues 495-504 [CMV], Influenza matrix protein 58-66 [FLU],], HTLV Tax 11-19 [HTLV] and HIV gag p17 76-84 [HIV]. Surface expression of constructs validated through fluorescence activated cell sorting (FACS) analysis monitoring mCherry proxy expression and anti-mCherry surface expression. Notably, subsequent to the generation of this figure an additional (5^{th}) control epitope was identified and added to test set encoding for EBV BMLF1 residues 259-267 [EBV]. The surface expression profile of EBV mirrors those observed for the other 4 controls (data not shown).

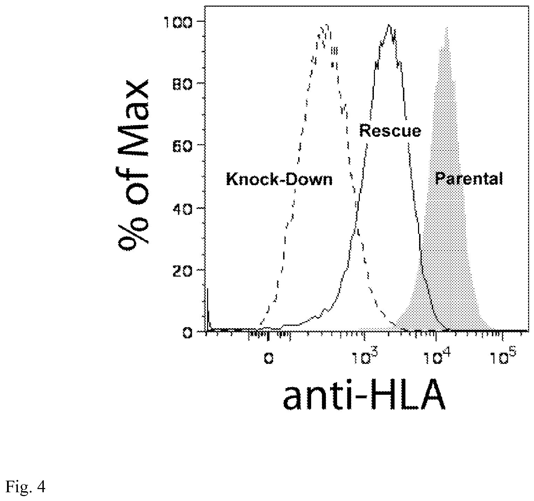

Figure 4. Validating proper folding and epitope presentation of the MHC controls. Here, plasma membrane surface staining of Class-I HLA:B2M complexes is shown using W6/32 anti-MHC-Class I mAb illustrating 1) endogenous expression of Class I MHC (labeled as Parental), 2) ∼95% knock down through lentivral delivery of shRNA targeting the 5' UTR of native human B2M (Knock-Down), and 3) rescue of Class-I MHC expression upon addition of our sc-p construct (Rescue). Notably, the construct does not contain the 5' UTR and thus is immune to shRNA down-regulation. The mAb W6/32 used requires that both MHC and B2M are properly folded and membrane localized for binding.

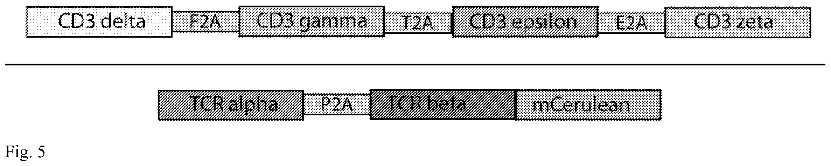

Figure 5. Constructs used for TCR expression and membrane localization in HEK cells. To allow for expression of control TCRs lentiviral co-transduction techniques were used, wherein one lentiviral construct harbors the full CD3 gene cassette (top) linked by various viral 2A peptides. The 2A "self-cleaving" peptides used were derived from the foot-and-mouth disease virus (F2A), *Thosea asigna* virus (T2A) and the equine rhinitis A virus (E2A). The second construct (bottom) carries the TCR alpha and beta chains linked by a viral porcine teschovirus-1 (P2A) peptide to allow for stoichiometric expression of each chain as this peptide shows the highest "cleavage" efficiency in mammalian cells [2]. The mCerulean (BLUE) expression proxy follows the beta chain transmembrane segment.

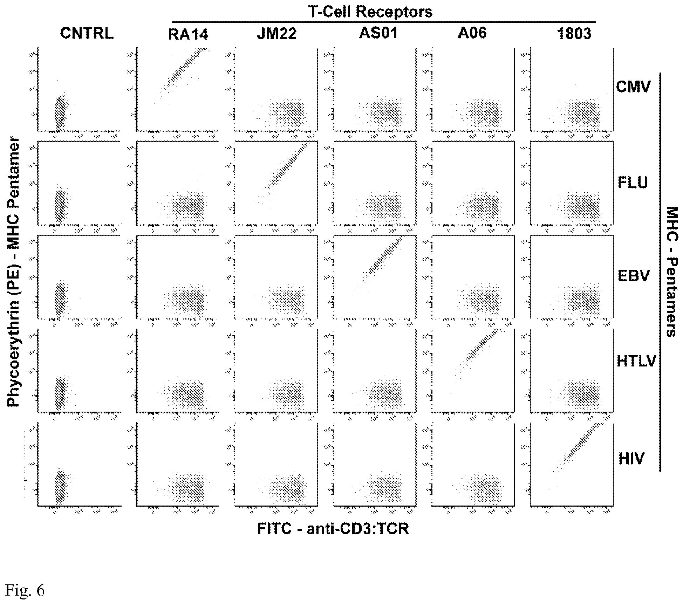

Figure 6. Surface expression of active hetero-dimeric TCR control constructs in HEK cells Proof-of-principle studies employ the 5 cognate TCRs for the HLA molecules discussed above (TCR RA14 [binds to CMV peptide], JM22 [FLU], AS01 [EBV], A06 [HTLV] and 1803 [HIV]). Surface expression and active T-cell complex formation confirmed through FACS analysis against surface anti-CD3 (FITC labeled, x-axis) and surface MHC pentamer staining (Phycoerythrin [PE], y-axis). Untransduced cells were used as a negative control (CNTRL).

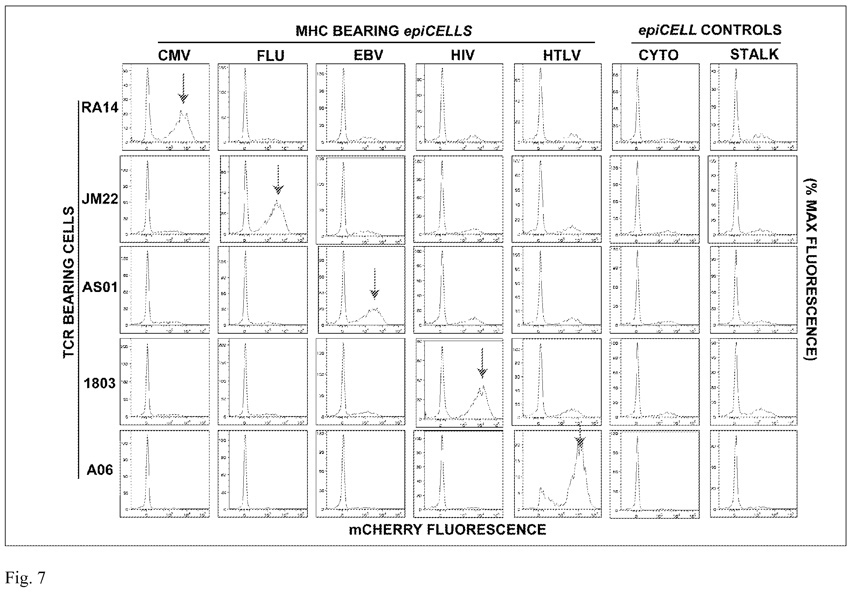

Figure 7. Arrayed cell-cell FACS analysis for Initial validation of the epiCELL platform. The 5 TCRs (RA14, JM22, ASO1, 1803, A06) were individually expressed to complement the 5 cognate sc-pMHC epiCELLS (CMV, FLU, EBV, HIV, HTLV). Cytoplasmic mCherry (CYTO) and surface expressed mCherry (without the MHC, STALK) were used as negative controls. Histograms from FACS analysis of the individual and mixed populations clearly demonstrated a significant increase (as much as 100-fold, A06:HTLV interaction) in signal representing specific cell-cell interactions only when cells expressing cognate MHC:TCR pairs were both present, and correlate with traditional pentamer challenge (Figure 6). Positive interactions are marked with red arrows.

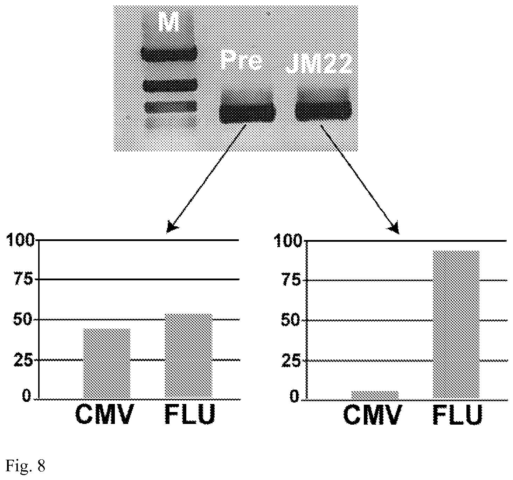

Figure 8. Results for the *epiCELL* platform. Two *epiCELL* constructs (CMV and FLU) were pooled, challenged with independent TCR bearing HEK cells (JM22), and sorted on the conjugates formed (using the mCherry surface expression proxy to track the *epiCELL* and mCerulean as the TCR expression proxy). (The fluorescent protein is not a required part of the construct when magnetic separation is being used, but is helpful for manual or lower throughput processes). The genomic DNA from each pool was extracted and subjected to ~30 cycles of PCR using universal primers targeting flanking regions around the epitope. The resulting PCR bands are shown (top) for the Pre-sorted *epiCELL* pool (labeled as Pre) and JM22 challenged sets. These amplicons were submitted for library preparation and subsequent next generation sequencing was performed on an illumina MiSeq platform. The sequencing files were analyzed and epitopes readily identified. For each, the absolute number of epitope sequences observed were counted and normalized as a percent of ALL observed NGS reads passing our QC filter, the labels represent the pathogenic epitope for CMV and FLU (bottom).

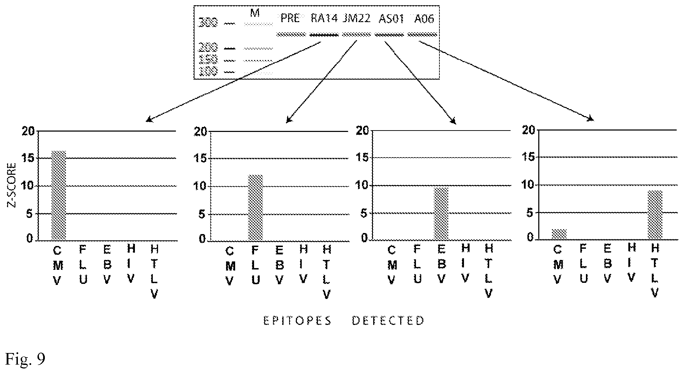

Fig. 9. Five *epiCELL* constructs (CMV, FLU, EBV, HIV, HTLV) were pooled, challenged with independent TCR-bearing HEK cells (R14, JM22, AS01, A06), and sorted on the conjugates formed (using the mCherry surface expression proxy to track the *epiCELL* and mCerulean as the TCR expression proxy). The genomic DNA from each pool was extracted and subjected to ~30 cycles of PCR using universal primers targeting flanking regions around the epitope. The bioanlyzer output for the resulting PCR bands are shown (top) for the Pre-sorted *epiCELL* pool (labeled as Pre) and TCR challenged sets. These amplicons were submitted for library preparation and subsequent next generation sequencing was performed on an illumina MiSeq platform. The sequencing files were analyzed and epitopes readily identified. Epitopes identified within the pre-sorted population (the library) was within a range from 16-23% (data not shown). For each of the TCR challenged data sets, the absolute number of epitope sequences observed were counted and normalized as a percent of all observed NGS reads that pass our QC filter and was used to calculate a Z-score.

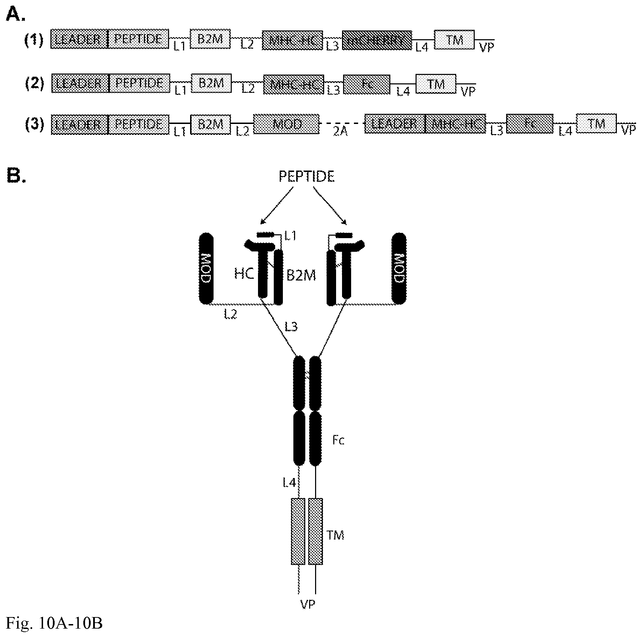

Fig. 10A-10B. Exemplary alternate surface expression constructs for use in epiCELL and its derivatives, e.g., viratope. The variants utilize a native human B2M leader sequence immediately followed by a candidate epitope (labeled as peptide) further coupled to the B2M molecule through linker L1. 10A: A(1) is analogous to a "traditional" epiCELL based presentation with the addition of a viral packaging signal at the extreme C-terminal end (e.g, GP41 env residues 706-713, etc., labeled as VP). To allow for bivalent display, an Fc Fusion based construction has been utilized A(2), again terminating in a VP packaging signal. In this instance, epitopes for traditional antibodies (e.g., FLAG, MYC, etc.) are placed in linker L4 to allow for surface detection. Lastly, to increase the modularity/flexibility of the epiCELL screening platform, synTac based expression constructs are utilized. synTac's split the MHC construct into respective heavy and light chains and fuse both peptides and proteins to various ends (e.g., construct A(3) and schematically represented in panel 10B). All components associate during production within eukaryotic cells (e.g., HEK, CHO) and self-assemble. Individual chains are covalently tethered through disulfide bridges (shown as RED lines). All constructs are held in the membrane through a native Class-I Heavy Chain transmembrane domain (TM).

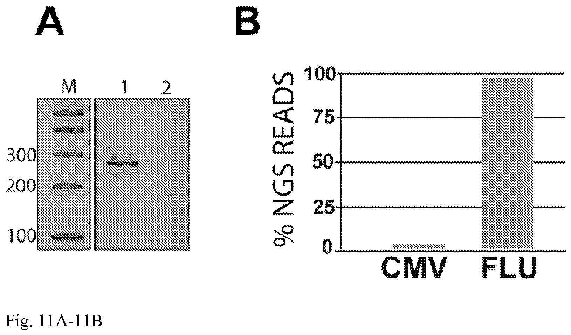

Fig. 11A-11B: RT-PCR and Next GEN Sequencing from viratope particle's. The genomic RNA from each viratope pool was extracted through lysis and subjected to one round of reverse transcription (RT, 42 degrees C for 20 minutes), followed by ∼30 cycles of PCR using universal primers targeting flanking regions around the epitope. The resulting PCR bands are shown in panel 11A. Notably, a PCR band is only observed in the presence of an initial RT step (lane 1) and is absent when RT is omitted (lane 2), supporting the generation of competent retrovirus derived from epiCELLS. These amplicons were submitted for next generation sequencing (NGS) and epitopes readily identified (Panel 11B).

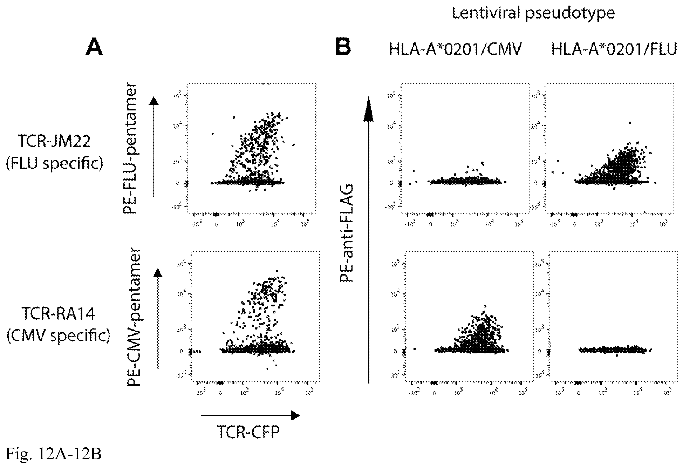

Fig. 12A-B: Viratope: lentiviral particles pseudotyped with peptide-HLA-A*0201 Fc fusion proteins for detection of antigen-specific T cell populations. Single chain constructs (Fig. 10A, No. 2) composed of a peptide epitope linked to beta-2 microglobulin, HLA-A*0201, and human IgG1 Fc were substituted for the envelope component of a third generation lentiviral transfection system. The constructs also contained a FLAG epitope tag for detection by secondary antibodies (placed in the L4 linker region). The peptide epitopes presented in the context of HLA-A*0201 were either the NLVPMVATV peptide epitope from human cytomegalovirus (CMV) or the GILGFVFTL peptide epitope from influenza (FLU). Harvested lentivirus was concentrated and applied to HEK cells previously transfected with either a specific or irrelevant T cell receptor (TCR). Excess lentivirus was washed from cells and the remaining cell-bound lentivirus was detected via a PE-conjugated anti-FLAG antibody. Lentivirus pseudotyped with the cognate, but not the irrelevant epitope bound to the respective cognate TCR-expressing HEK cells in a manner comparable to staining by specific peptide-MHC pentamers.

### DETAILED DESCRIPTION OF THE INVENTION

A (i) lentivirus-like particle or (ii) lentivirus is provided in which lentivirus-like particle or a lentivirus is physically associated with an expression product via a mammalian cell membrane portion associated with the lentivirus-like particle or lentivirus and the mammalian transmembrane domain of the expression product, the expression product comprising in N-terminal to C-terminal order:
a 5 to 20 amino acid peptide, contiguous with
a first linker peptide sequence, contiguous with
a beta 2 microglobulin sequence, contiguous with
a second linker peptide sequence, contiguous with
a class I Major Histocompatibility Complex (MHC) heavy chain sequence, contiguous with
a third linker peptide sequence, contiguous with
a fluorescent protein or a sequence of an immunoglobulin Fc domain, contiguous with
a fourth linker peptide sequence, contiguous with
the mammalian transmembrane domain, contiguous with
a viral packaging sequence comprising the GP41 envelop protein residues 706-713.

An isolated suspension-adapted mammalian cell expressing an expression product of a heterologous nucleic acid transduced or transfected therein, or a membrane-bound portion of such cell expressing the expression product, which expression product comprises, in N-terminal to C-terminal order:
a 5 to 20 amino acid peptide, contiguous with
a first linker peptide sequence, contiguous with
a beta 2 microglobulin sequence, contiguous with
a second linker peptide sequence, contiguous with
a class I Major Histocompatibility Complex heavy chain sequence, contiguous with
a third linker peptide sequence, contiguous with
a fluorescent protein or a sequence of an immunoglobulin Fc domain, contiguous with
a fourth linker peptide sequence, contiguous with
a mammalian transmembrane domain, contiguous with
a lentiviral packaging sequence comprising the GP41 envelop protein residues 706-713.

In aspects regarding cells and constructs discussed herein comprising an immunoglobulin Fc domain, the immunoglobulin Fc domain can have the sequence of a human Ig Fc, preferably a human IgG1 Fc. In another embodiment, such immunoglobulin Fc domain can have the sequence of a murine IgG2a Fc. Notably, where there are expressed constructs each comprising an immunoglobulin Fc domain, spontaneous bivalent fusion may occur. Accordingly, the discussed transduced or transfected cells, membrane-bound portions of such expressing the expression products as well as virus-like particles and viruses as described herein may demonstrate bivalent fusion of the expressed immunoglobulin Fc domains.

In an aspect of the encoded, or the expressed, 5 to 20 amino acid peptides described herein, the peptide is one of 5, 6, 7, 8, 9, 10, 11, 12, 13, 14, 15, 16, 17, 18, 19 or 20 amino acids in length. In an embodiment the peptide is 8, 9, 10, 11, or 12 amino acids in length. In an embodiment the peptide is a nonamer (i.e. 9 amino acids in length). The sequence can be preselected as desired.

In an aspect of the cell, and of the other cells and constructs discussed herein comprising a mammalian transmembrane domain, the transmembrane domain has the sequence of a mammalian transmembrane domain but is not taken from a mammal itself, for example it is a sequence engineered to have an identical or similar sequence to a mammalian transmembrane domain protein sequence. In an aspect, the sequence is the same as a mammalian MHC transmembrane sequence. In an aspect, the sequence is the same as a Major Histocompatibility Complex heavy chain transmembrane domain. In an aspect, the sequence is the same as a Class I Major Histocompatibility Complex heavy chain transmembrane domain. MHC I alpha 3 sequences are known in the art. In an aspect, the sequence is the same as a human Class I Major Histocompatibility Complex heavy chain transmembrane domain. As used herein, "contiguous with" in regard to two nucleotide sequences means the first sequence is consecutive with the second sequence via, for example, a phosphodiester bond. As used herein, "contiguous with" in regard to two peptide/oligopeptide sequences means the first sequence is consecutive with the second sequence via, for example, a peptide bond.

Any nucleic acid-encoded fluorescent proteins are usable in the invention described herein. Such proteins are well-known in the art. Non-limiting examples include a GFP, RFP, YFP, mFRUIT, mPlum, mCherry, tdTomato, mStrawberry, J-Red, DsRed-monomer, mOrange, mKO, mCitrine, Venus, YPet, EYFP, Emerald, EGFP, CyPet, mCFPm, Cerulean, and T-Sapphire.

A suspension-adapted cell is one that is able to survive or proliferate in a suspension culture. A heterologous nucleic acid is one that is heterologous relative to the cell into which it is transfected or transduced, the heterologous nucleic acid as a whole not naturally existing in the cell prior to transfection or transduction.

Linker sequences are short peptide sequences, including short repeat peptide sequences, known in the art. They generally do not interfere with or have minimal functional impact on other encoded peptide functions of the domains or regions they link. For example, a linker can be 4 repeats of GGGGS for one or more linker(s). Linkers as described herein, apart from the specific exception of the self-cleaving linker as referred to herein are stable in that they are not-self cleaving. With regard to the exception referred to, the self-cleaving linker, a non-limiting example of such is a viral P2A peptide, which peptides shows good self-cleaving efficiency in mammalian cells.

In an aspect of the isolated suspension-adapted cell, the cell is transduced by or transfected with a heterologous nucleic acid comprising, in 5' to 3' order:
a leader oligonucleotide sequence, contiguous with
an oligonucleotide sequence encoding a 5 to 20 amino acid peptide, contiguous with
an oligonucleotide sequence encoding a first linker, contiguous with
an oligonucleotide sequence encoding a beta 2 microglobulin sequence, contiguous with
an oligonucleotide sequence encoding a second linker, contiguous with
an oligonucleotide sequence encoding a Major Histocompatibility Complex heavy chain sequence, contiguous with
an oligonucleotide sequence encoding a third linker, contiguous with
an oligonucleotide sequence encoding an immunoglobulin Fc domain, contiguous with
an oligonucleotide sequence encoding a fourth linker, contiguous with
an oligonucleotide sequence encoding a mammalian transmembrane domain.

In an aspectt of the isolated suspension-adapted cell, the cell is transduced by or transfected with a heterologous nucleic acid comprising, in 5' to 3' order:
a leader oligonucleotide sequence, contiguous with
an oligonucleotide sequence encoding a 5 to 20 amino acid peptide, contiguous with
an oligonucleotide sequence encoding a first linker, contiguous with
an oligonucleotide sequence encoding a beta 2 microglobulin sequence, contiguous with
an oligonucleotide sequence encoding a second linker, contiguous with
an oligonucleotide sequence encoding a Major Histocompatibility Complex heavy chain sequence, contiguous with
an oligonucleotide sequence encoding a third linker, contiguous with
an oligonucleotide sequence encoding a fluorescent protein, contiguous with
an oligonucleotide sequence encoding a fourth linker, contiguous with
an oligonucleotide sequence encoding a mammalian transmembrane domain.

Also described is an isolated suspension-adapted cell expressing an expression product of a heterologous nucleic acid transduced or transfected therein, or a membrane-bound portion of such cell expressing the expression product, which expression product comprises, in N-terminal to C-terminal order:
a 5 to 20 amino acid peptide, contiguous with
a first linker peptide sequence, contiguous with
a beta 2 microglobulin sequence, contiguous with
a second linker peptide sequence, contiguous with
a Major Histocompatibility Complex heavy chain sequence, contiguous with
a third linker peptide sequence, contiguous with
a fluorescent protein or a sequence of an immunoglobulin Fc domain, contiguous with
a fourth linker peptide sequence, contiguous with
a mammalian transmembrane domain.

In an embodiment, the membrane-bound portion expressing the expression product of the cell is provided. In an embodiment, the membrane-bound portion is a microvessicle or a exosome.

In an embodiment, the cell expresses the expression product comprising the sequence of the immunoglobulin Fc domain.

The disclosure also provides the cell as described, or the membrane-bound portion, except wherein a linker thereof, such as a fourth linker, is additionally connected to a fluorescent protein (such as, for example, an mCherry) or an epitopes for a known antibodies (e.g., FLAG, MYC) as proxy for surface expression. In an aspect, the linker of the cell as described, or the membrane-bound portion does not comprise such and is only a linker (for example as described elsewhere herein).

In an aspect of the isolated suspension-adapted cell, or membrane-bound portion of such cell expressing the expression product, the cell expresses the expression product comprising the fluorescent protein.

In an aspect of the isolated suspension-adapted cell, or membrane-bound portion of such cell expressing the expression product, the cell expresses the expression product comprising the immunoglobulin Fc domain.

In an aspect of the isolated suspension-adapted cell, or membrane-bound portion of such cell expressing the expression product, the mammalian transmembrane domain is a Major Histocompatibility Complex heavy chain transmembrane domain.

In an aspect, the heterologous nucleic acid further comprises an oligonucleotide encoding a viral packaging sequence 3' relative to the oligonucleotide sequence encoding the mammalian transmembrane domain.

In an aspect of the isolated suspension-adapted cell, or of the membrane-bound portion of such cell expressing the expression product, the expression product further comprises a viral packaging sequence that is C-terminal relative to the mammalian transmembrane domain.

In an aspect of the transduced cells, recombinant nucleic acids, or heterologous nucleic acids described herein that encode a viral packaging sequence, the relevant nucleic acid can be, in an embodiment, an RNA sequence. In an aspect, the viral packaging sequence is a retroviral viral packaging sequence.

In an aspect, a membrane-bound portion expressing the expression product of the cell as described herein is provided, and is a viral like particle.

In an aspect of the isolated suspension-adapted cells as described herein or the membrane-bound portion of such cells expressing the expression product, the beta 2 microglobulin has the same sequence as a human beta 2 microglobulin.

In an aspect of the isolated suspension-adapted cells as described herein or the membrane-bound portion of such cells expressing the expression product, the Major Histocompatibility Complex heavy chain sequence has the same sequence as a human HLA-A sequence.

In an aspect of the isolated suspension-adapted cells as described herein or the membrane-bound portion of such cells expressing the expression product, the transmembrane domain has the same sequence as a human Major Histocompatibility Complex I heavy chain transmembrane domain.

Also provided is a plurality of the isolated suspension-adapted cells as described. Also provided is a plurality of membrane-bound portions of such cells expressing the expression product, wherein the plurality comprises at least two different encoded 5 to 20 amino acid peptides.

In an aspect of the pluralities, the plurality comprises at least 100 different encoded 5 to 20 amino acid peptides.

Also provided is the isolated suspension-adapted cell as described or membrane-bound portion of such cell expressing the expression product, or the plurality of the isolated suspension-adapted cells or membrane-bound portions of described, wherein the encoded peptide(s) is a nonamer or are nonamers. In an aspect the encoded 5-20 amino acid peptide or peptides is or are presented on an extracellular surface of the cells.

Also provided is a membrane-bound portion of an isolated suspension-adapted cell as described expressing the expression product.

Also provided is an isolated suspension-adapted cell as described.

In an aspect of the isolated suspension-adapted cell(s), the heterologous nucleic acid encodes the viral packaging sequence and the cell is transduced by or transfected with a virus, plasmid or viral vector comprising the heterologous nucleic acid.

Also provided is a (i) lentivirus-like particle or (ii) lentivirus, produced by the transduced or transfected cell as described herein, which virus like particle or a virus is physically associated via a cell membrane portion having attached thereto, by a mammalian transmembrane domain, an expression product comprising in N-terminal to C-terminal order:
a 5 to 20 amino acid peptide, contiguous with
a first linker peptide sequence, contiguous with
a beta 2 microglobulin sequence, contiguous with
a second linker peptide sequence, contiguous with
a class I Major Histocompatibility Complex heavy chain sequence, contiguous with
a third linker peptide sequence, contiguous with
a fluorescent protein or a sequence of an immunoglobulin Fc domain, contiguous with
a fourth linker peptide sequence, contiguous with
the mammalian transmembrane domain, contiguous with
a lentiviral packaging sequence comprising the GP41 envelop protein residues 706-713.

In an aspect of the (i) virus-like particle or (ii) virus, the retroviral transfection system comprises a packaging plasmid having therein, in place of an oligonucleotide sequence or sequences encoding one or more envelope proteins, an oligonucleotide sequence or sequences encoding a 5 to 20 amino acid peptide, contiguous with
a first linker peptide sequence, contiguous with
a beta 2 microglobulin sequence, contiguous with
a second linker peptide sequence, contiguous with
a Major Histocompatibility Complex heavy chain sequence, contiguous with
a third linker peptide sequence, contiguous with
a fluorescent protein or an immunoglobulin Fc domain, contiguous with
a fourth linker peptide sequence, contiguous with
the mammalian transmembrane domain.

Also described is an isolated virus, which virus has budded from the cell as described herein. Budded viruses take with them, or are associated with, a portion of the membrane of the cell and as such are associated with the expressed membrane located constructs described herein.

Also described is an isolated virus-like particle has budded from the cell as described herein. Budded virus-like particles take with them, or are associated with, a portion of the membrane of the cell and as such are associated with the expressed membrane located constructs described herein.

Also provided is a plurality of the isolated lentiviruses as described herein. In an embodiment, the plurality comprises viruses which differ in the encoded 5 to 20 amino acid peptides thereof.

Also provided is a plurality of the isolated lentivirus-like particles as described herein. In an embodiment, the plurality comprises lentivirus-like particles which differ in the encoded 5 to 20 amino acid peptides thereof.

In an embodiment of the lentiviruses, the expressed recombinant polypeptide comprises the fluorescent protein. In an embodiment of the lentiviruses, the expressed recombinant polypeptide comprises the immunoglobulin Fc domain. In an embodiment of the lentivirus-like particles, the expressed recombinant polypeptide comprises the fluorescent protein. In an embodiment of the lentivirus-like particles, the expressed recombinant polypeptide comprises the immunoglobulin Fc domain.

Also described is a recombinant nucleic acid comprising, in 5' to 3' order:
a sequence encoding a leader oligonucleotide sequence, contiguous with
an oligonucleotide sequence encoding a 5 to 20 amino acid peptide, contiguous with
an oligonucleotide sequence encoding a first linker, contiguous with
an oligonucleotide sequence encoding a beta 2 microglobulin sequence, contiguous with
an oligonucleotide sequence encoding a second linker, contiguous with
an oligonucleotide sequence encoding a Major Histocompatibility Complex heavy chain sequence, contiguous with
an oligonucleotide sequence encoding a third linker peptide sequence, contiguous with
an oligonucleotide sequence encoding a fluorescent protein or an immunoglobulin Fc domain, contiguous with
an oligonucleotide sequence encoding a fourth linker peptide sequence, contiguous with
an oligonucleotide sequence encoding a mammalian transmembrane domain.

In an aspect, the recombinant nucleic acid comprises the oligonucleotide sequence encoding the fluorescent protein. In an aspect, the recombinant nucleic acid comprises the oligonucleotide sequence encoding the immunoglobulin Fc domain. In an aspect, the mammalian transmembrane domain is a Major Histocompatibility Complex heavy chain transmembrane domain. In an aspect, the mammalian transmembrane domain has the same sequence is a mammalian HLA-A*0201 domain. In an aspect, the HLA-A*0201 is human.

In an aspect, the recombinant nucleic acid further comprises an oligonucleotide encoding a viral packaging sequence 3' relative to the oligonucleotide sequence encoding the mammalian transmembrane domain. In an aspect, the recombinant nucleic acid is a vector. In an aspect, the recombinant nucleic acid is a viral vector. In an aspect, the recombinant nucleic acid is a retroviral vector. In an aspect, the recombinant nucleic acid is a lentiviral vector. In an aspect, the recombinant nucleic acid vector is a plasmid.

In an , of the recombinant nucleic acid or of the isolated suspension-adapted cells, or the heterologous or recombinant nucleic acid comprises cDNA.

Also described is an isolated suspension-adapted cell transduced by or transfected with a heterologous nucleic acid comprising, in 5' to 3' order:
an oligonucleotide sequence encoding a first B2M leader sequence,
contiguous with an oligonucleotide sequence encoding a preselected 5 to 20 amino acid peptide, contiguous with an oligonucleotide sequence encoding a first amino acid linker sequence, contiguous with an oligonucleotide sequence encoding a sequence of amino acids identical to a human native B2M peptide sequence,
contiguous with an oligonucleotide sequence encoding a second amino acid linker sequence,
contiguous with an oligonucleotide sequence encoding a preselected second peptide sequence,
contiguous with an oligonucleotide sequence encoding a third amino acid linker,
contiguous with an oligonucleotide sequence encoding a second B2M leader sequence,
contiguous with an oligonucleotide sequence encoding a sequence of amino acids identical to a MHC heavy chain,
contiguous with an oligonucleotide sequence encoding a fourth amino acid linker,
contiguous with an oligonucleotide sequence encoding a sequence of amino acids identical to an immunoglobulin Fc domain,
contiguous with an oligonucleotide sequence encoding a fifth linker,
contiguous with an oligonucleotide sequence encoding a mammalian transmembrane domain.

In an aspect, the preselected second peptide sequence is an immune system effector molecule. In an aspect, the preselected second peptide sequence is a detectable epitope. In non-limiting examples the detectable epitope is a FLAG epitope or a MYC epitope. In an aspect, the preselected second peptide sequence is a fluorescent protein, as described herein. In an aspect the preselected second peptide sequence can be a naturally occurring or synthetic affinity reagent targeting, e.g., a cell surface glycan or other post-translational modification (e.g., sulfation). Examples include, but are not limited to, members of the TNF/TNFR family (OX40L, ICOSL, FASL, LTA, LTB TRAIL, CD153, TNFSF9, RANKL, TWEAK, TNFSF13, TNFSF13b, TNFSF14, TNFSF15, TNFSF18, CD40LG, CD70) or affinity reagents directed at the TNF/TNFR family members; members of the Immunoglobulin superfamily (VISTA, PD1, PD-L1, PDL2, B71, B72, CTLA4, CD28, TIM3, CD4, CD8, CD19, T cell receptor chains, ICOS, ICOS ligand, HHLA2, butyrophilins, BTLA, B7-H3, B7-H4, CD3, CD79a, CD79b, IgSF, CAMS including CD2, CD58, CD48, CD150, CD229, CD244, ICAM-1), Leukocyte immunoglobulin like receptors (LILR), killer cell immunoglobulin like receptors (KIR)), lectin superfamily members, selectins, cytokines/chemokine and cytokine/chemokine receptors, growth factors and growth factor receptors), adhesion molecules (integrins, fibronectins, cadherins), or ecto-domains of multi-span intergral membrane protein, or affinity reagents directed at the Immunoglobulin superfamily and listed gene products. In addition, active homologs/orthologs of these gene products, including but not limited to, viral sequences (e.g., CMV, EBV), bacterial sequences, fungal sequences, eukaryotic pathogens (e.g., Schistosoma, Plasmodium, Babesia, Eimeria, Theileria, Toxoplasma, Entamoeba, Leishmania, and trypanosoma), and mammalian -derived coding regions. In an aspect the preselected second peptide sequence can be a T cell stimulatory domain or can be a T cell inhibitory domain. In an aspect the preselected second peptide sequence can be cell surface protein ectodomain.

Also described is an isolated suspension-adapted cell expressing an expression product of a heterologous nucleic acid transduced or transfected therein, or a membrane-bound portion of such cell expressing the expression product, which expression product comprises,
a recombinant polypeptide construct comprising (i) a preselected 5 to 20 amino acid peptide bound by a first amino acid linker sequence contiguous with a sequence of amino acids comprising a sequence identical to a human native B2M peptide sequence contiguous with a second amino acid linker sequence contiguous with a preselected second peptide sequence, wherein (i) is bound by one, or more than one, disulfide bond to (ii) a sequence of amino acids having the sequence of a MHC heavy chain contiguous with a fourth amino acid linker
sequence contiguous with a sequence of amino acids identical to an immunoglobulin Fc domain contiguous with a fifth amino acid linker, contiguous with a mammalian transmembrane domain. In aspects, the preselected second peptide sequence, and the other components, are as recited elsewhere herein.

Also described is an isolated suspension-adapted cell transduced by or transfected with a virus, plasmid or viral vector comprising a heterologous nucleic acid comprising, in 5' to 3' order:
an oligonucleotide sequence encoding a first B2M leader sequence,
contiguous with an oligonucleotide sequence encoding a preselected 5 to 20 amino acid peptide, contiguous with an oligonucleotide sequence encoding a first amino acid linker sequence, contiguous with an oligonucleotide sequence encoding a sequence of amino acids identical to a human native B2M peptide sequence,
contiguous with an oligonucleotide sequence encoding a second amino acid linker sequence,
contiguous with an oligonucleotide sequence encoding a preselected second peptide sequence,
contiguous with an oligonucleotide sequence encoding a third amino acid linker,
contiguous with an oligonucleotide sequence encoding a second B2M leader sequence,
contiguous with an oligonucleotide sequence encoding a sequence of amino acids identical to a MHC heavy chain,
contiguous with an oligonucleotide sequence encoding a fourth amino acid linker,
contiguous with an oligonucleotide sequence encoding a sequence of amino acids identical to an immunoglobulin Fc domain,
contiguous with an oligonucleotide sequence encoding a fifth linker,
contiguous with an oligonucleotide sequence encoding a mammalian transmembrane domain, contiguous with an oligonucleotide encoding a viral packaging sequence. Viral packaging sequences or signals are known in the art and are also described herein. In an embodiment, the third amino acid linker is self-cleaving after expression. Self-cleaving linkers are described herein, such as the viral P2A peptide.

Also described is a (i) virus like particle or (ii) virus, produced by the instant cell, which virus like particle or a virus is physically associated via a cell membrane portion having attached thereto, by a mammalian transmembrane domain, an expression product comprising in N-terminal to C-terminal order:
a recombinant polypeptide construct comprising (i) a preselected 5 to 20 amino acid peptide bound by a first amino acid linker sequence contiguous with a sequence of amino acids comprising a sequence identical to a human native B2M peptide sequence contiguous with a second amino acid linker sequence contiguous with a preselected second peptide sequence,
wherein (i) is bound by one, or more than one, disulfide bond to (ii) a sequence of amino acids having the sequence of a MHC heavy chain contiguous with a fourth amino acid linker sequence contiguous with a sequence of amino acids identical to an immunoglobulin Fc domain contiguous with a fifth amino acid linker, contiguous with a mammalian transmembrane domain, contiguous with a viral packaging sequence. In an aspect of the (i) virus like particle or (ii) virus, the mammalian transmembrane domain is a Major Histocompatibility Complex heavy chain transmembrane domain. In an aspect, the heterologous nucleic acid further comprises an oligonucleotide encoding a viral packaging sequence 3' relative to the oligonucleotide sequence encoding the mammalian transmembrane domain. In an aspect of the (i) virus like particle or (ii) virus, the preselected second peptide is a T Cell modulatory domain, an antibody epitope, a fluorescent protein, a nucleic acid binding protein or a comodulatory protein.

Also described is a recombinant nucleic acid comprising, in 5' to 3' order:
an oligonucleotide sequence encoding a first B2M leader sequence,
contiguous with an oligonucleotide sequence encoding a preselected 5 to 20 amino acid peptide, contiguous with an oligonucleotide sequence encoding a first amino acid linker sequence, contiguous with an oligonucleotide sequence encoding a sequence of amino acids identical to a human native B2M peptide sequence,
contiguous with an oligonucleotide sequence encoding a second amino acid linker sequence,
contiguous with an oligonucleotide sequence encoding a preselected second peptide sequence,
contiguous with an oligonucleotide sequence encoding a third amino acid linker,
contiguous with an oligonucleotide sequence encoding a second B2M leader sequence,
contiguous with an oligonucleotide sequence encoding a sequence of amino acids identical to a MHC heavy chain,
contiguous with an oligonucleotide sequence encoding a fourth amino acid linker,
contiguous with an oligonucleotide sequence encoding a sequence of amino acids identical to an immunoglobulin Fc domain,
contiguous with an oligonucleotide sequence encoding a fifth linker,
contiguous with an oligonucleotide sequence encoding a mammalian transmembrane domain.

In an aspect of the recombinant nucleic acid, the mammalian transmembrane domain is a Major Histocompatibility Complex heavy chain transmembrane domain. In an aspect of the recombinant nucleic acid, the recombinant nucleic acid further comprises an oligonucleotide encoding a viral packaging sequence 3' relative to the oligonucleotide sequence encoding the mammalian transmembrane domain .

Also described is a method of identifying a T-cell epitope comprising contacting a T-cell with a plurality of isolated suspension-adapted cells comprising at least two cells, or a membrane-bound portion of such cells expressing the expression product, each cell or membrane bound portion expressing an expression product of a heterologous nucleic acid transduced or transfected therein, each of which expression products comprises a 5 to 20 amino acid peptide, contiguous with first linker peptide sequence, contiguous with a beta 2 microglobulin sequence, contiguous with a second linker peptide sequence, contiguous with a Major Histocompatibility Complex heavy chain sequence, contiguous with a third linker peptide sequence, contiguous with a fluorescent protein or an immunoglobulin Fc domain, contiguous with a fourth linker peptide sequence, contiguous with a mammalian transmembrane domain, wherein the plurality of isolated suspension-adapted cells or membrane-bound portions expresses at least two different encoded 5 to 20 amino acid peptides among the cells or membrane-bound portions under conditions permitting T-cells to conjugate with the 5 to 20 amino acid peptides;
recovering T-cell(s) which have formed a conjugate with a suspension-adapted cell or membrane-bound portions;
recovering DNA from the suspension-adapted cell(s);
sequencing the recovered DNA;
identifying the 5 to 20 amino acid peptide(s) encoded for in the DNA,
so as to thereby identify a T-cell epitope.

In an aspect of recovering the T-cells in the methods described herein, the conjugate is recovered.
Also described is a method of identifying a T-cell epitope comprising contacting a T-cell with a plurality of isolated suspension-adapted cells comprising at least two cells, or membrane-bound portions thereof, each expressing an expression product of a heterologous nucleic acid transduced or transfected therein, each of which expression products comprises (i) a preselected 5 to 20 amino acid peptide bound by a first amino acid linker sequence contiguous with a sequence of amino acids comprising a sequence identical to a human native B2M peptide sequence contiguous with a second amino acid linker sequence contiguous with a preselected second peptide sequence,
wherein (i) is bound by one, or more than one, disulfide bond to (ii) a sequence of amino acids having the sequence of a MHC heavy chain contiguous with a fourth amino acid linker
sequence contiguous with a sequence of amino acids identical to an immunoglobulin Fc
domain contiguous with a fifth amino acid linker, contiguous with a mammalian transmembrane domain, wherein the plurality of isolated suspension-adapted cells or membrane-bound portions thereof expresses at least two different encoded 5 to 20 amino acid peptides among the cells or portions under conditions permitting T-cells to conjugate with the 5 to 20 amino acid peptides;
recovering T-cell(s) which have formed a conjugate with a suspension-adapted cell or membrane-bound portion;
recovering DNA from the suspension-adapted cell(s);
sequencing the recovered DNA;
identifying the 5 to 20 amino acid peptide(s) encoded for in the DNA,
so as to thereby identify a T-cell epitope.

In an aspect of the methods, the T-cell(s) which have formed a conjugate are recovered by flow cytometry. In an aspect of the methods, the T-cell(s) which have formed a conjugate are recovered by fluorescence activated cell sorting.

In an aspect of the methods, the method comprises amplifying the recovered DNA prior to sequencing. In an aspect of the methods, the amplifying is effected using one or more universal primers. In an aspect of the methods, one or more of the universal primers is directed to a portion of the sequence of the heterologous nucleic acid but is not complementary to a nucleic acid encoding a native beta 2 microglobulin sequence of the cell.

In an aspect of the methods, the mammalian transmembrane domain is a Major Histocompatibility Complex heavy chain transmembrane domain. In an aspect of the methods, the T-cells comprise peripheral T-cells obtained from a subject. In an aspect of the methods, the subject is human.

In an aspect of the methods, the method further comprises identifying any of the 5-20 amino acid peptides encoded for in the DNA that are enriched in the recovered DNA relative to their presence in the DNA of the plurality of isolated suspension-adapted cells, so as to thereby identify one or more immunodominant T-cell epitope(s).

In an aspect of the methods, the methods further comprise comparing the level of the T cell conjugate with a level of control which is a recombinantly engineered T cell receptor (TCR)-expressing control cell, and wherein levels in excess of control indicate an immunodominant epitope.

In an aspect of the methods, the isolated suspension-adapted cell is a mammalian cell. In an aspect, the isolated suspension-adapted cell is an HEK cell.

In an aspect of the methods, the isolated suspension-adapted cells are employed. In an aspect of the methods, the isolated membrane-bound portions of the cells expressing the expression product suspension-adapted cells are employed.

Also described is a method of identifying a T-cell epitope comprising
contacting a T-cell with a plurality of isolated suspension-adapted cells comprising at least two cells, or virus-like particle or viruses associated with a membrane portion of such cells, the cells or membrane bound portion expressing an expression product of a heterologous nucleic acid transduced or transfected therein, each of which expression products comprises a 5 to 20 amino acid peptide, contiguous with first linker peptide sequence, contiguous with a beta 2 microglobulin sequence, contiguous with a second linker peptide sequence, contiguous with a Major Histocompatibility Complex heavy chain sequence, contiguous with a third linker peptide sequence, contiguous with a fluorescent protein or an immunoglobulin Fc domain, contiguous with a fourth linker peptide sequence, contiguous with a mammalian transmembrane domain, contiguous with a viral packaging sequence, wherein the plurality of isolated suspension-adapted cells or of virus-like particles or viruses associated with the membrane portion of the cells, expresses at least two different encoded 5 to 20 amino acid peptides among the cells or virus-like particles or viruses under conditions permitting T-cells to conjugate with the 5 to 20 amino acid peptides;
recovering T-cell(s) which have formed a conjugate with a suspension-adapted cell, virus-like particle or virus of the plurality;
recovering DNA from the suspension-adapted cell or RNA from the virus-like particle or virus;
sequencing the recovered DNA or RNA;
identifying the 5 to 20 amino acid peptide(s) encoded for in the DNA or RNA,
so as to thereby identify a T-cell epitope.

Also described is a method of identifying a T-cell epitope comprising contacting a T-cell with a plurality of isolated suspension-adapted cells comprising at least two cells, or virus-like particles or viruses associated with a membrane portion of such cells, each expressing an expression product of a heterologous nucleic acid transduced or transfected therein, each of which expression products comprises (i) a preselected 5 to 20 amino acid peptide bound by a first amino acid linker sequence contiguous with a sequence of amino acids comprising a sequence identical to a human native B2M peptide sequence contiguous with a second amino acid linker sequence contiguous with a preselected second peptide sequence,
wherein (i) is bound by one, or more than one, disulfide bond to (ii) a sequence of amino acids having the sequence of a MHC heavy chain contiguous with a fourth amino acid linker sequence contiguous with a sequence of amino acids identical to an immunoglobulin Fc domain contiguous with a fifth amino acid linker, contiguous with a mammalian transmembrane domain, wherein the plurality of isolated suspension-adapted cells, virus-like particles or viruses expresses at least two different encoded 5 to 20 amino acid peptides among the cells thereof under conditions permitting T-cells to conjugate with the 5 to 20 amino acid peptides;
recovering T-cell(s) which have formed a conjugate with a suspension-adapted cell, virus-like particle or virus associated membrane portion;
recovering DNA from the suspension-adapted cell or RNA from the virus-like particle or virus;
sequencing the recovered DNA or RNA;
identifying the 5 to 20 amino acid peptide(s) encoded for in the DNA or RNA,
so as to thereby identify a T-cell epitope.

In an aspect, the virus-like particles or viruses associated with a membrane portion of the cell have budded from such cells.

In an aspect of the methods, the T-cell(s) which have formed a conjugate are recovered by flow cytometry. In an aspect of the methods, the T-cell(s) conjugates are recovered via FACS or secondary antibody staining methods. In an aspect, the secondary antibody is directed to a preselected second peptide sequence.

In an aspect the methods comprise amplifying the recovered DNA or RNA prior to sequencing. In an aspect, the amplifying is effected using one or more universal primers.

In an aspect, the T-cell(s) which have formed a conjugate are recovered by (i) contacting the T-cell(s) which have formed a conjugate with a magnetic bead having attached to an external surface thereof an antibody or antibody fragment directed against a T-cell surface marker molecule and (ii) applying a magnetic field to the beads so as to recover the magnetic beads.

In an aspect, the T-cell surface marker molecule is a CD8 molecule.

In an aspect of the methods, the suspension adapted cells or membrane portions thereof express the fluorescent protein and T-cell(s) which have formed a conjugate are recovered by fluorescence activated cells sorting based on fluorescence of said fluorescent protein.

In an aspect of the methods, the virus like particles or viruses are recovered by a secondary antibody-based system, wherein the secondary antibody is directed to an epitope in the expressed construct. Non-limiting examples of such epitopes include FLAG and MYC epitopes.

In an aspect of the inventions described, isolated suspension-adapted cell, the beta 2 microglobulin has the same sequence as a human beta 2 microglobulin. In an aspect of the isolated suspension-adapted cell, the Histocompatibility Complex heavy chain sequence has the same sequence as a human HLA-A sequence. In an aspect of the isolated suspension-adapted cell, the Histocompatibility Complex heavy chain transmembrane domain has the same sequence as a human Major Histocompatibility Complex I heavy chain transmembrane domain

In an aspect of the inventions described, the pluralities can comprises at least 100 different encoded 5-20 amino acid peptides. In an aspect, the peptides are 8, 9, 10, 11 or 12 amino acid peptides. In an aspect, the plurality comprises at least 1000 different encoded 8, 9, 10, 11 or 12 amino acid peptides. In an aspect, the plurality comprises at least 10,000 different encoded 8, 9, 10, 11 or 12 amino acid peptides. In an aspect, the plurality comprises at least 100,000 different encoded 8, 9, 10, 11 or 12 amino acid peptides. In an aspect, the plurality comprises at least 1 x 10⁶ different encoded 8, 9, 10, 11 or 12 amino acid peptides. In an aspect, the plurality comprises at least 1 x 10⁷ different encoded 8, 9, 10, 11 or 12 amino acid peptides. In an aspect, the plurality comprises at least 1 x 10⁸ different encoded 8, 9, 10, 11 or 12 amino acid peptides.

In an example of the isolated suspension-adapted cell, or of the plurality of the isolated suspension-adapted cells, the encoded peptide is a nonamer (9 amino acids in length).

In an example of the disclosure, the encoded peptide is presented on an extracellular surface of the cells.

In an aspect, the recombinant nucleic acid is a vector. In an aspect, the vector is a viral vector. In an aspect, the viral vector is a lentiviral vector.

In an aspect of the isolated suspension-adapted cells, of the plurality of the isolated suspension-adapted cells, or of the recombinant nucleic acid, the nucleic acid comprises DNA.

In an aspect, one or more of the universal primers is directed to a portion of the sequence of the heterologous nucleic acid but is not complementary to a nucleic acid encoding a native beta 2 microglobulin sequence of the cell.

In an aspect, the T-cells comprise peripheral T-cells obtained from a subject.

In an aspect of the methods herein, the subject is human.
In an aspect, the method comprises comparing results obtained to those for a recombinantly engineered TCR-expressing control cell. In an aspect, the recombinantly engineered TCR-expressing control cell is an HEK cell.

In an aspect of the cells, the beta 2 microglobulin has the same sequence as a human beta 2 microglobulin. In an aspect, the Histocompatibility Complex heavy chain sequence has the same sequence as a human HLA-A sequence. In an embodiment, the Histocompatibility Complex heavy chain transmembrane domain has the same sequence as a human Major Histocompatibility Complex I heavy chain transmembrane domain

A plurality of the isolated suspension-adapted cells is provided, wherein the plurality comprises at least two different encoded 8, 9, 10, 11 or 12 amino acid peptides.

In the context of isogenic cell lines (single integration per cell) the practical limit is equal to the complexity of the library used. In other words, scaled based on number of cells in the reaction - for example 10⁶ - 10⁸.

In an embodiment of the invention, the linker between the beta 2 microglobulin and the Major Histocompatibility Complex heavy chain can be removed resulting in two separate products. These will assemble naturally in the cell.

In an embodiment, the nucleic acid comprises the following sequence: atgtctcgctccgtggccttagctgtgctcgcgctactctctctttctggcctggaggcc**(n)ₓ**ggtggaggtggttctggaggaggc ggttcgggcggaggtggtagtatccagcgtactccaaagattcaggtttactcacgtcatccagcagagaatggaaagtcaaatttcc tgaattgctatgtgtctgggtttcatccatccgacattgaagttgacttactgaagaatggagagagaattgaaaaagtggagcattcag acttgtctttcagcaaggactggtctttctatctcttgtattatactgaattcacccccactgaaaaagatgagtatgcctgccgtgtgaac cacgtgactttgtcacagcccaagatagttaagtgggatcgagacatgggaggcggaggatctggtggtggaggttctggtggtgg gggatctggctctcactccatgaggtatttcttcacatccgtgtcccggcccggccgcggggagccccgcttcatcgcagtgggcta cgtggacgacacgcagttcgtgcggttcgacagcgacgccgcgagccagaggatggagccgcgggcgccgtggatagagcag gagggtccggagtattgggacggggagacacggaaagtgaaggcccactcacagactcaccgagtggacctggggaccctgcg cggcgcctacaaccagagcgaggccggttctcacaccgtccagaggatgtatggctgcgacgtggggtcggactggcgcttcctc cgcgggtaccaccagtacgcctacgacggcaaggattacatcgccctgaaagaggacctgcgctcttggaccgcggcggacatg gcagctcagaccaccaagcacaagtgggaggcggcccatgtggcggagcagttgagagcctacctggagggcacgtgcgtgga gtggctccgcagatacctggagaacgggaaggagacgctgcagcgcacggacgcccccaaaacgcatatgactcaccacgctg tctctgaccatgaagccaccctgaggtgctgggccctgagcttctaccctgcggagatcacactgacctggcagcgggatgggga ggaccagacccaggacacggagctcgtggagaccaggcctgcaggggatggaaccttccagaagtgggcggctgtggtggtgc cttctggacaggagcagagatacacctgccatgtgcagcatgagggtttgcccaagcccctcaccctgagatgggagccgggtgg aggcggatctggcggcggaggatctggaggaggtggatctgggggcggtggtagtggcctgaatgacatctttgaagcccagaa aatcgaatggcacgaaatggtgagcaagggcgaggaggataacatggccatcatcaaggagttcatgcgcttcaaggtgcacatg gagggctccgtgaacggccacgagttcgagatcgagggcgagggcgagggccgcccctacgagggcacccagaccgccaag ctgaaggtgaccaagggtggccccctgcccttcgcctgggacatcctgtcccctcagttcatgtacggctccaaggcctacgtgaag caccccgccgacatccccgactacttgaagctgtccttccccgagggcttcaagtgggagcgcgtgatgaacttcgaggacggcg gcgtggtgaccgtgacccaggactcctccctccaggacggcgagttcatctacaaggtgaagctgcgcggcaccaacttcccctcc gacggccccgtaatgcagaagaagacaatgggctgggaggcctcctccgagcggatgtaccccgaggacggcgccctgaagg gcgagatcaagcagaggctgaagctgaaggacggcggccactacgacgctgaggtcaagaccacctacaaggccaagaagcc cgtgcagctgcccggcgcctacaacgtcaacatcaagttggacatcacctcccacaacgaggactacaccatcgtggaacagtac gaacgcgccgagggccgccactccaccggcggcatggacgagctgtacaagggtggaggtggttctggaggaggcggttcga gcagccagccgaccattccgattgtgggcattattgcgggcctggtgctgtttggcgcggtgattaccggcgcggtggtggcggcg gtgatgtggcgtcgtaaaagcagcgatcgtaaagattataaagatgatgatgataaataatag (SEQ ID NO:1), wherein (n)ₓ is an 8, 9, 10, 11 or 12 amino acid-encoding nucleotide sequence, with x being 24, 27, 30, 33, or 36 nucleotides, respectively. In an embodiment, the 24, 27, 30, 33, or 36 nucleotides are comprised of 8, 9, 10, 11, or 12 codons, or equivalents, respectively.

The recombinant nucleic acid may be up to ∼3000 nt for lentiviral delivery. The recombinant nucleic acid may be up to ∼10,000 nt for plasmid delivery.

In an embodiment, the nucleic encodes, or the expression product comprises, the following sequence:
MSRSVALAVLALLSLSGLEAX₍ₙ₎GGGGSGGGGSGGGGSIQRTPKIQVYSRHPAENGKSNFLNCY VSGFHPSDIEVDLLKNGERIEKVEHSDLSFSKDWSFYLLYYTEFTPTEKDEYACRVNHVTLSQPK IVKWDRDMGGGGSGGGGSGGGGSGSHSMRYFFTSVSRPGRGEPRFIAVGYVDDTQFVRFDSDA ASQRMEPRAPWIEQEGPEYWDGETRKVKAHSQTHRVDLGTLRGAYNQSEAGSHTVQRMYGCD VGSDWRFLRGYHQYAYDGKDYIALKEDLRSWTAADMAAQTTKHKWEAAHVAEQLRAYLEG TCVEWLRRYLENGKETLQRTDAPKTHMTHHAVSDHEATLRCWALSFYPAEITLTWQRDGEDQ TQDTELVETRPAGDGTFQKWAAWVPSGQEQRYTCHVQHEGLPKPLTLRWEPGGGGSGGGGS GGGGSGGGGSGLNDIFEAQKIEWHEMVSKGEEDNMAIIKEFMRFKVHMEGSVNGHEFEIEGEG EGRPYEGTQTAKLKVTKGGPLPFAWDILSPQFMYGSKAYVKHPADIPDYLKLSFPEGFKWERV MNFEDGGVVTVTQDSSLQDGEFIYKVKLRGTNFPSDGPVMQKKTMGWEASSERMYPEDGALK GEIKQRLKLKDGGHYDAEVKTTYKAKKPVQLPGAYNVNIKLDITSHNEDYTIVEQYERAEGRHS TGGMDELYKGGGGSGGGGSSSQPTIPIVGIIAGLVLFGAVITGAVVAAVMWRRKSSDRKDYKD DDK (SEQ ID NO:2), wherein X₍ₙ₎ is a 8, 9, 10, 11, or 12 amino acid peptide sequence. ₍ₙ₎ can be any one of 8, 9, 10, 11, or 12 or the range or a sub-range thereof.

A leader sequence includes any signal peptide that can be processed by a mammalian cell. Such sequences are well-known in the art.

Fluorescent proteins usable in the invention include GFP, RFP, YFP, mFRUIT. Any nucleic acid-encodable fluorescent protein may be used, for example mPlum, mCherry, tdTomato, mStrawberry, J-Red, DsRed-monomer, mOrange, mKO, mCitrine, Venus, YPet, EYFP, Emerald, EGFP, CyPet, mCFPm, Cerulean, T-Sapphire, GFP.

An exemplary non-limiting B2M Leader is MSRSVALAVLALLSLSGLEA (SEQ ID NO:3).

An exemplary non-limitin B2M sequence is


 (SEQ ID NO:4).

An exemplary non-limiting MHC Heavy Chain sequence is

GSHSMRYFFTSVSRPGRGEPRFIAVGYVDDTQFVRFDSDAASQRMEPRAPWIEQEG
PEYWDGETRKVKAHSQTHRVDLGTLRGAYNQSEAGSHTVQRMYGCDVGSDWRF
LRGYHQYAYDGKDYIALKEDLRSWTAADMAAQTTKHKWEAAHVAEQLRAYLEG
TCVEWLRRYLENGKETLQRTDAPKTHMTHHAVSDHEATLRCWALSFYPAEITLTW
QRDGEDQTQDTELVETRPAGDGTFQKWAAVVVPSGQEQRYTCHVQHEGLPKPLT
LRWEP (SEQ ID NO:5).

An exemplary non-limiting immunoglobulin Fc Domain sequence is

DKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAP
IEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSL
SPGK (SEQ ID NO:6).

An exemplary non-limiting viral packaging (VP) sequence (signal) is NRVRQGYS (SEQ ID NO:7). The viral packaging sequence may be 8 to 20 amino acids in length. In one example, the viral packaging sequence is 8 amino acids in length.

In one example, the non-cleaving linkers are each, independently, from 5 to 40 amino acids in length. In one example, the non-cleaving linkers are each, independently, from 5 to 30 amino acids in length. In one example, the non-cleaving linkers are each, independently, from 5 to 20 amino acids in length. In one example, the non-cleaving linkers are each 20 amino acids in length.

As used herein, having the "same sequence" means having 95% or greater sequence similarity with the referenced sequence without preventing the established or known function of the reference sequence. In an embodiment, having the same sequence means having a sequence completely identical to the referenced sequence.

All combinations of the various elements described herein are within the scope of the invention unless otherwise indicated herein or otherwise clearly contradicted by context.

This invention will be better understood from the Experimental Details, which follow. However, one skilled in the art will readily appreciate that the specific methods and results discussed are merely illustrative of the invention as described more fully in the claims that follow thereafter.

### EXPERIMENTAL DETAILS

Herein is described a novel mammalian cell display platform for the presentation of candidate T-cell epitopes ("epiCELL") for high throughput T-cell epitope mapping from patient samples (immunomonitoring), preferably using highly sensitive and massively parallel next-generation sequencing as the readout.

The approach centers on the use of a novel membrane-anchored single chain peptide MHC (sc-pMHC) mammalian cell display platform to allow for the presentation of large numbers of T-cell epitopes in the context of class I MHC on the surface of, e.g., HEK cells. These expression pools are challenged with T-cells from, for example, healthy, infected, cured and immunized patients to identify those epitopes that are directly relevant to disease, treatment and neutralization, for, in a non-limiting example, category A-C pathogens. Immunodominant signatures identified from the pathogens can be assembled into a pathogen epitope collection for use as a rapid and portable diagnostic tool, with detection occurring directly from whole blood.

The preferred strategy exploits a library of sc-pMHC constructs displayed on the surface of mammalian cells and challenged against patient/cohort specific peripheral T-cells to directly identify disease relevant epitopes. As illustrated in Fig. 1, the library of sc-pMHC vectors can be pooled and transfected (or transduced in the case of, e.g., lentiviral pools) *en masse* into suspension adapted cells, such as HEK293 cells, thus generating an epiCELL pool.

### Example 1

The epiCELL pool is mixed with patient derived peripheral T-cells (purified from whole blood samples using standard protocols [26]) and allowed to form conjugates through the specific engagement of TCRs with their cognate sc-pMHC ligands expressed by the epiCELL pool. Conjugates are then recovered by, for example, magnetic separation for the processing of multiple patient samples in parallel, or more traditional flow cytometric sorting procedures for single samples. The epitope sequences from the enriched pool members are amplified by PCR (using universal primers) and subjected to next-generation deep sequencing to identify epitopes enriched by the capture process. These enriched epitopes directly identify immunodominant T-cell epitopes. Further subsequent validation, if desired, can be effected by *in vitro* methods (e.g. cytokine ELISpot, FACS) [27]. The strategy allows for the rapid identification of all disease relevant immunodominant epitopes from a single patient sample. Notably, this approach can be multiplexed through the use of indexed adapters, e.g. TruSeq®, to vastly increase throughput and reduce costs (e.g., multiple patient samples can be run on a single lane of an NGS flow cell).

The Construct: One overall design for the membrane anchored class I sc-pMHC molecule is presented in Fig. 2. Briefly, this construct utilizes a native human B2M leader sequence to allow for plasma membrane localization immediately followed by a candidate epitope (labeled as peptide). Once in the ER the leader sequence is fully removed and allows for the presentation of the peptide in the MHC binding pocket. This is further coupled to the native B2M molecule, the human HLA-A02:01 allele, and a surface exposed mCherry expression proxy through linker regions (4 repeats of GGGGS (SEQ ID NO:8) for each linker). The entire construct is held in the membrane through a native Class-I Heavy Chain transmembrane domain (HC TM). The covalent linkage of antigenic peptides to the MHC class-I molecule is well established and has been highly effective both *in vitro* and *in vivo* [28-30]. This strategy eliminates difficulties associated with unintended T-cell binding/activation that arise from the exchange of non-covalent peptide complexes (cross-presentation), especially those that are weak binding. Furthermore, this modular design is readily amenable to high-throughput molecular biology manipulations (e.g., cloning/sequencing) for the generation (and subsequent interrogation) of large libraries coding for distinct peptide sequences within the context of a given MHC allele. This platform leverages the "universal" nature of the single chain construction. In this paradigm, the only difference amongst the library is the 27-nucleotide sequence encoding the 9-mer-peptide itself (e.g., the epitope). This unique sequence can be amplified with "universal primers" (labeled as Forward/Reverse in Figure 2) and readily identified by deep sequencing and subsequent translation following T-cell challenge [31-34]. Notably, the nucleotide sequence forming the consensus region for "universal" primer annealing has been modified away from the native B2M nucleotide sequence to avoid background amplification from the endogenous B2M pool (i.e. B2M from the HEK cell).

MHC controls. Initial feasibility studies within our group leverage 5 known pathogenic HLA-A02 restricted epitopes linked to 5 independent viral pathogens (cytomegalovirus pp65 protein residues 495-504 [henceforth referred to as CMV], Influenza matrix protein 58-66 [FLU], Epstein-Barr virus BMLF1 259-267 [EBV], Human T-lymphotropic virus Tax 11-19 [HTLV] and HIV gag p17 76-84 [HIV]). HEK293 stable cell lines were generated by lentiviral transduction of virus carrying sc-pMHC constructs bearing a surface mCherry expression proxy anchored to the membrane through the native human class-I heavy chain transmembrane domain as illustrated in Figure 2. Surface expression of our constructs was validated through fluorescence-activated cell sorting (FACS) analysis monitoring mCherry proxy expression and anti-mCherry surface expression (4 of which are illustrated in Figure 3), and supports that the constructs express and are properly targeted to the plasma membrane. Notably, the EBV control peptide was added subsequent to the generation of this figure, however the surface expression profile of EBV mirrors those observed for the other 4 controls (data not shown).

In this system, surface presentation of mCherry is an indicator of proper folding of the MHC construct, as unfolded proteins are more often trapped/retained in the ER/Golgi [35], however a direct assessment on MHC folding is of course desirable. As HEK293 cells natively express HLA and B2M molecules, direct staining against surface B2M or HLA to monitor proper folding is challenging. To ensure that the single chain membrane anchored MHC design results in properly folded material shRNA hairpins targeting the 5' untranslated region (UTR) of native human B2M (pGIPZ clone VA282 [catalogue number RHS4430-101098345], knock-down cells provided by the Einstein shRNA core facility) were leveraged to down regulate the endogenous expression of B2M and surface MHC within HEK cells (B2M was chosen for down regulation as it is essential for MHC folding/localization regardless of MHC isotype). This implementation does not contain the 5' UTR to promote persistent expression of the integrated constructs. As shown in Figure 4, greater than 95% of native HLA was effectively down-regulated using the shRNA strategy as monitored by surface staining against a conformationally dependent anti-Class I MHC antibody (the mAb W6/32 used requires that both MHC and B2M are properly folded for binding [36]. Notably proper processing of the leader peptide and epitope presentation are a requirement of MHC stability/folding). MHC surface expression was restored upon transduction with a representative construct from the library (CMV). Taken together, this suggests that the constructs are both properly localized (as monitored by anti-mCherry) and well folded (as monitored by anti-HLA, Figure 4).

TCR controls. Given the extensive use of suspension adapted HEK cell lines within this lab, HEK cells were naturally chosen as the expression host for generation of control TCR lines. HEK 293 cells do not endogenously express TCR genes, nor are they capable of expressing TCR constructs without modification (as observed by ourselves [data not shown] and others [37]). The TCR is a disulfide-linked membrane-anchored heterodimer (alpha/beta chains) expressed as part of a complex with the invariant CD3 chain molecules. The CD3 chains, together with the TCR, form what is known as the T-cell receptor complex. The full complex is required for proper expression and plasma membrane localization. To allow for expression of control TCRs, lentiviral co-transduction techniques were utilized, wherein one lentiviral construct harbors the full CD3 gene cassette linked by various viral 2A "self-cleaving" peptides [37] (Figure 5, top) and the second carries the TCR alpha and beta chains linked by a single viral P2A peptide to allow for stoichiometric expression as the P2A peptide shows the highest "cleavage" efficiency in mammalian cells [2]. The mCerulean (BLUE) expression proxy follows the beta chain transmembrane segment (Figure 5, bottom). Proof-of-principle studies employ the 5 cognate TCRs for the HLA molecules discussed above (TCR RA14 [binds to CMV peptide], JM22 [FLU], AS01 [EBV], A06 [HTLV] and 1803 [HIV]). Surface expression of the constructs was confirmed by anti-TCR as well as anti-CD3 antibody staining (data not shown) and active T-cell complex formation confirmed through cognate MHC pentamer staining (Figure 6, pentamers purchased from ProImmune). Untransduced cells were used as a negative control.

The epiCELL screening platform. While traditional MHC tetramer- (or the more recent pentamer-) based presentation affords enhanced avidity relative to single proteins, the expression of the query protein on the plasma membrane of eukaryotic cells is expected to provide greater antigen density, significantly higher avidity and expanded dynamic range for detecting weaker pMHC:TCR interactions. The 5 TCRs were individually expressed to complement the 5 cognate sc-pMHC epiCELLS. Cytoplasmic mCherry (labeled as CYTO) and surface expressed mCherry (without the MHC, labeled as STALK) were used as negative controls. Flow cytometric analysis of the individual and mixed populations clearly demonstrated a significant increase (as much as 100-fold, A06:HTLV interaction) in signal representing specific cell-cell interactions only when cells expressing cognate MHC:TCR pairs were both present (Figure 7). Next two of the epiCELLs (CMV and FLU) were pooled, challenged with independent TCR bearing HEK cells (JM22 only), and sorted on the conjugates formed. The genomic DNA from each pool was extracted and subjected to ∼30 cycles of PCR using universal primers targeting flanking regions around the epitope (Figure 2, above). The resulting PCR bands are shown in Figure 8 (top), these amplicons were submitted for library preparation (e.g., addition of multiplexed TruSeq indexed adapters) and subsequent next generation sequencing (NGS) was performed (illumina MiSeq). Library preparation and sequencing was performed at the Einstein Epigenomics core facility. The resulting FASTQ files from the NGS run were analyzed and epitopes readily identified (Figure 8, bottom). For each, the absolute number of epitope sequences observed were counted and normalized as a percent of ALL observed NGS reads that pass our QC filter. Notably, CMV and FLU was selected for initial screening as these were the first validated constructs in the library.

In a further example, an exhaustive screen against all overlapping 9-mers representing the EBV BMLF1 protein (a pool of ∼400 epiCELLS) can be surveyed to identify immunodominant signatures from EBV infected patients (this target was chosen as ∼95% of adults >35 years old maintain EBV reactive peripheral T-cells).

The epiCELL platform cab be used in defining the entire ensemble of biologically relevant T-cell epitopes associated with human disease. Next-generation-sequencing (NGS)-based epiCELL platform for epitope mapping can be combined with larger "combinatorial" libraries to allow for extension to mimotope screening against select TCRs to identify binders with altered affinities/kinetics, and further extended to the survey of all immunologic reactivities within a single patient sample simultaneously and with a sensitivity approaching comprehensive coverage. The identification of peptide antigen epitopes (and mimotopes) is an important first step in identifying, isolating and modulating class I MHC restricted T-cells involved in protective and pathological immune responses. Notably, the methods described can easily be extended/modified to an analogous exhaustive survey of Class-II (CD4+ T-cell) reactivities.

### Example 2

In this experiment, lentiviruses for all 5 MHC bearing *epiCELLS* (CMV, FLU, EBV, HIV, HTLV) were transduced separately, cells pooled in equal ratios and challenged against 4 cognate TCR bearing cells (RA14, JM22, AS01, A06) independently and sorted on the conjugates formed. The genomic DNA from each pool was extracted and subjected to ∼30 cycles of PCR using universal primers targeting flanking regions around the epitope. The resulting PCR bands are shown in Figure 9 (top), these amplicons were submitted for library preparation (e.g., addition of multiplexed TruSeq® indexed adapters) and subsequent next generation sequencing (NGS) was performed (Illumina MiSeq). Library preparation and sequencing was performed at the Albert Einstein College of Medicine epigenomics core facility. The resulting FASTQ files from the NGS run were analyzed and epitopes readily identified (Figure 9, bottom). Epitopes identified within the pre-sorted population (the library) was within a range from 16-23% (data not shown). For each of the TCR challenged data sets, the absolute number of epitope sequences observed were counted and normalized as a percent of all observed NGS reads that pass the QC filter and was used to calculate a Z-score using the mean and standard deviation parameters taken from the pre-sorted pool. These results highlight the utility of the epiCELL platform and show the robustness of the screen even using un-optimized binding/washing protocols.

### Example 3

The utility of *epiCELL* can be extended to include not only single chain constructs (with and without bivalent presentation through Fc fusion), but also split constructs (synTacs) to allow for local presentation of multiple protein or peptide fragments within the context of *epiCELLs.* Furthermore, the use of small plasma membrane containing fragments derived from *epiCELL* pools (e.g., microvessicles, exosomes, viral like partices [VLPs] and retroviruses [e.g., lentivirus, etc.]) have allowed for vast decreases in reaction volumes for screening (these viral particle-based approaches are sometimes referred to herein as *"viratopes"*)*.* These expression pools (*epiCELLS* or *viratopes*) are challenged with T-cells from healthy, infected, cured and immunized patients to identify those epitopes that are directly relevant to disease, diagnosis, treatment, neutralization and monitoring of disease progression and therapeutic response.

Exemplary variants continue to utilize a sequence the same as a native human B2M leader sequence immediately followed by a candidate peptide epitope that is covalently linked to the B2M molecule through linker L1 (illustrated in Fig. 10A). However, the 3 new constructs differ in their overall architecture and covalent organization. A first variant (See Fig. 10A.(1)) is analogous to traditional *epiCELL* based presentation, but with the addition of a viral packaging signal at the extreme C-terminal end (e.g, GP41 envelope protein residues 706-713, etc., labeled as VP). The presence of the VP sequence has no consequence in the context of traditional *epiCELL* based screening, but does allow for packaging into viral like particles (VLPs) or retroviruses (e.g., lentivirus) when budded from *epiCELL* pools. To increase local valency of surface expressed constructs, an Fc-Fusion-based construction is used (Fig. 10A. (2), e.g., human IgG1 Fc, murine IgG2a Fc, etc.), again terminating in a VP sequence to allow for viral packaging. As the proxy for surface expression (i.e., mCherry) has been removed from this variant, epitopes for traditional antibodies (e.g., FLAG, MYC, etc.) have been placed in linker L4 to allow for detection of plasma membrane localization via antibody staining. These currently described constructs (A. 1 and A.2) have utilized a single chain construction and as such are limited with respect to the ability to extend the system through alternative protein linkages for screening purposes. To increase the modularity/flexibility of the epiCELL screening platform through the inclusion of additional protein or peptide linkages, a synTac-based expression construct, also developed in this laboratory, is utilized. Briefly, the strategy underlying synTac splits the MHC construct into respective heavy and light chains, with fusion of both peptides and proteins to various termini (Fig. 10A. (3)) and schematically represented Fig. 10B). This construction results in covalent fusion of the peptide epitope to the N-terminus of the light chain (B2M) followed by a carboxy terminal extension of the light chain to our MOD effector molecule, Fig. 10B. In this scenario the heavy chain (HLA-molecule) is fused to the Fc region. All components associate during production within eukaryotic cells (e.g., HEK, CHO) and self-assemble. Notably, the two chains are covalently tethered through disulfide bonds (shown as RED lines). The MOD in this case can be any protein, peptide or other chemical entity required for screening. Examples are antibody epitopes (FLAG, MYC etc.) for secondary staining, fluorescent proteins (GFP, mFruit, etc.) for direct fluorescent detection, nucleic acid binding proteins or comodulatory proteins. All constructs are localized to the plasma membrane through a native Class-I Heavy Chain transmembrane domain (TM, although this could be any human, murine or other TM domain). Universal primers are used to amplify the unique 27-nucleotide sequence (9-mer peptide) found in a current construct following T-cell challenge to directly identify disease relevant epitopes. However, peptides of lengths varying from, but not restricted to, 5-20 amino acids are candidate epitopes.

Extending the epiCELL screening platform for lentiviral display: *Viratope.* In the context of full combinatorial screening (e.g., when the peptide sequence is fully randomized), volumes ensuring full library coverage (i.e., 10⁹ epitopes) can range from 10-100 milliliters when using traditional *epiCELLs.* This requirement results predominantly from the relatively large size of the HEK cells used for *epiCELL* display, coupled with poor cellular viability at high concentrations (e.g., at concentrations greater than 10 million per ml). Retroviruses (e.g., lentivirus) are routinely generated from HEK cells through transfection with a packaging plasmid that contains specific virus-encoded genes (termed helper plasmid), along with an envelope protein (VSV-G, etc.). Notably, retroviral particles budded from these cells are stable at extreme concentrations (greater than 1 billion per mL). To take advantage of decreased reaction volumes and enhanced stability, we leveraged the viral packaging signals (VP) within our surface displayed MHC constructs to allow for packing into viral particles following transfection of *epiCELL* pools with helper plasmid alone (no addition of envelope plasmid), effectively pseudotyping the budded lentivirus with peptide MHC. Specifically, single chain constructs (Fig. 10A. (2)) composed of a peptide epitope linked to beta-2 microglobulin (B2M), HLA-A*0201, and human IgG1 Fc were substituted for the envelope component of a third generation lentiviral transfection system. The constructs also contained a FLAG epitope tag for detection by secondary antibodies (placed in the L4 linker region). The peptide epitopes presented in the context of HLA-A*0201 were either the NLVPMVATV (SEQ ID NO:9) peptide epitope from human cytomegalovirus (CMV) or the GILGFVFTL (SEQ ID NO:10) peptide epitope from influenza (FLU). Harvested lentivirus were concentrated 100x by ultracentrifugation and stored at 4 degrees Celsius. The genomic RNA (as opposed to DNA) from each viratope pool was extracted and subjected to one round of reverse transcription (RT) followed by ∼30 cycles of PCR using universal primers targeting flanking regions around the epitope. The resulting PCR bands are shown in Fig. 11A. Notably, a PCR band is only observed in the presence of an initial RT step (lane 1) and is absent when reverse transcriptase is omitted (lane 2), demonstrating the generation of competent (e.g., RNA-loaded) retrovirus. These amplicons were submitted for library preparation (e.g., addition of multiplexed TruSeq indexed adapters) and subsequent next generation sequencing (NGS) was performed (illumina MiSeq) to ensure compatibility of the process with *epiCELL* screening. Library preparation and sequencing was performed at the Einstein Epigenomics core facility. The resulting FASTQ files from the NGS run were analyzed and epitopes readily identified (Fig. 11B). Analogous to previous *epiCELL* binding experiments, *viratope* was then applied to HEK cells previously transfected with either a specific or irrelevant T cell receptor (TCR). Excess *viratope* particles were washed from cells and the remaining cell-bound lentivirus was detected via a PE-conjugated anti-FLAG antibody. *Viratope* pseudotyped with the cognate, but not the irrelevant epitope, bound to their respective TCR-expressing HEK cells in a manner comparable to staining by specific peptide-MHC pentamers (Fig. 12), demonstrating the specificity and general utility of *viratope* particles derived from *epiCELL* pools for epitope screening.

The current next-generation-sequencing (NGS) based *epiCELL*/*viratope* platform for epitope mapping can be combined with larger "combinatorial" libraries to allow for extension to mimotope screening against select TCRs to identify binders with altered affinities/kinetics, and further extended to the survey of ALL immunologic reactivities within a single patient sample simultaneously and with a sensitivity approaching comprehensive coverage. The identification of peptide antigen epitopes (and mimotopes) is important in identifying, isolating and modulating class I MHC restricted T-cells involved in protective and pathological immune responses. The methods described above are readily extended to an analogous survey of Class-II (CD4⁺ T-cell) reactivities.

### REFERENCES

1. Robins HS, S.S., Campregher PV, Turtle CJ, Andriesen J, Riddell SR, Carlson CS, Warren EH, Overlap and effective size of the human CD8+ T cell receptor repertoire. Sci Transl Med, 2010. 2(47): p. 47ra64.
2. Jin Hee Kim, S.-R.L., Li-Hua Li, Hye-Jeong Park, Jeong-Hoh Park, Kwang Youl Lee, Myeong-Kyu Kim,Boo Ahn Shin,Seok-Yong Choi, High Cleavage Efficiency of a 2A Peptide Derived from Porcine Teschovirus-1 in Human Cell Lines, Zebrafish and Mice. PlosOne, 2011. 6(4): p. e18556.
3. Wen F, Z.H., Construction and screening of an antigen-derived peptide library displayed on yeast cell surface for CD4+ T cell epitope identification. Methods Mol Biol, 2013. 1061: p. 245-64.
4. Yibing Wang, A.R., Ryan Heiser, Janice White, Frances Crawford, Philippa Marrack, and John W. Kappler, Using a baculovirus display library to identify MHC class I mimotopes. PNAS, 2004. 102(7): p. 2476-2481.
5. SJ, R., Peptide libraries for T cell epitope screening and characterization. J Immunol Methods, 2002. 267(1): p. 71-7.
6. Tim Beißbarth, J.A.T.-D., Gordon K. Smyth, Terence P. Speed, and Robert P. Anderson, A systematic approach for comprehensive T-cell epitope discovery using peptide libraries. Bioinformatics, 2005. 21: p. i29-i37.
7. Verena Rubio-Godoy, V.r.D., Yingdong Zhao, Richard Simon,Philippe Guillaume,Richard Houghten,Pedro Romero, Jean-Charles Cerottini,Clemencia Pinilla, and Danila Valmori, Positional Scanning-Synthetic Peptide Library-Based Analysis of Self- and Pathogen-Derived Peptide Cross-Reactivity with Tumor-Reactive Melan-A-Specific CTL. J Immunol, 2002. 169: p. 5696-5707.
8. Gokhan Deviren, K.G., Michael E. Paulaitis and Jonathan P. Schneck, Detection of antigen-specific T cells on p/MHC microarrays. J. Mol. Recognit, 2007. 20: p. 32-38.
9. Cox, A., Skipper, J, Chen, Y, Henderson, RA, Darrow, TL, Shabanowitz, J, Engelhard, VH, Hunt, DF, Slingluff, CL Jr, Identification of a peptide recognized by five melanomaspecific human cytotoxic T cell lines. Science, 1994. 264: p. 716-719.
10. Gerald T. Nepom, J.D.L., Forest M. White Susan Masewicz, Jarrod A. Marto, Andrew Herman, C. John Luckey, Ben Falk, Jeffrey Shabanowitz, Donald F. Hunt, Victor H. Engelhard, and Barbara S. Nepom, Identification and modulation of a naturally processed T cell epitope from the diabetes- associated autoantigen human glutamic acid decarboxylase 65 (hGAD65). PNAS, 2001. 98(4): p. 1763-1768.
11. Stern, L., Characterizing MHC-associated peptides by mass spectrometry. J Immunol., 2002. 2007(179): p. 2667-2668.
12. Evan W Newell, N.S., Nitya Nair,Brian A Kidd, Harry B Greenberg, Mark M Davis, Combinatorial tetramer staining and mass cytometry analysis facilitate T-cell epitope mapping and characterization. Nature Biotechnology, 2013. 31: p. 623-629.
13. Katherine S. Garmin, J.R.N.a.A.P., Genomic strategies for personalized cancer therapy. Hum. Mol. Genet., 2007. 16(R2): p. R226-R232.
14. Andrew H Sims, K.R.O., Robert B Clarke,and Anthony Howell, High-throughput genomic technology in research and clinical management of breast cancer. Exploiting the potential of gene expression profiling: is it ready for the clinic? Breast Cancer Research, 2006. 8(5): p. 214-221.
15. Muhammed Murtaza, S.-J.D., Dana W. Y. Tsui, Davina Gale, Tim Forshew, Anna M. Piskorz, Christine Parkinson, Suet-Feung Chin, Zoya Kingsbury, Alvin S. C. Wong, Francesco Marass, Sean Humphray, James Hadfield, David Bentley, Tan Min Chin, James D. Brenton, Carlos Caldas & Nitzan Rosenfeld, Non-invasive analysis of acquired resistance to cancer therapy by sequencing of plasma DNA. Nature, 2013. 497: p. 108-112.
16. Espina V, D.K., Cowherd S, Petricoin EF 3rd, Liotta LA, Use of proteomic analysis to monitor responses to biological therapies. Expert Opin Biol Ther. , 2004. 4(1): p. 83-93.
17. Moulay A. Alaoui-Jamali, D.P., Proteomic Profiling of Tyrosine Kinases as Pharmacological Endpoints for Targeted Cancer Therapy. Cancer Proteomics, ed. S.S. Daoud. 2008: Humana Press.
18. Daniel K. Nomura, M.M.D.B.F.C., Activity-based protein profiling for biochemical pathway discovery in cancer. Nature Reviews Cancer, 2010. 10: p. 630-638.
19. Donal J. Brennan, D.P.O.C., Elton Rexhepaj, Fredrik Ponten & William M. Gallagher, Antibody-based proteomics: fast-tracking molecular diagnostics in oncology. Nature Reviews Cancer, 2010. 10: p. 605-617.
20. TD., R., The current status and future potential of personalized diagnostics: Streamlining a customized process. Biotechnol Annu Rev, 2008. 14: p. 411-422.
21. Robins, H., Immunosequencing: applications of immune repertoire deep sequencing. Current Opinion in Immunology, 2013. 25: p. 1-7.
22. FA., W., TNF inhibition as therapy for rheumatoid arthritis. Expert Opin Investig Drugs, 2002. 11(7): p. 947-953.
23. Mansh, M., Ipilimumab and cancer immunotherapy: a new hope for advanced stage melanoma. Yale J Biol Med, 2011. 84(4): p. 381-389.
24. Larsen CP, P.T., Adams AB, Tso P, Shirasugi N, Strobert E, Anderson D, Cowan S, Price K, Naemura J, Emswiler J, Greene J, Turk LA, Bajorath J, Townsend R, Hagerty D, Linsley PS, Peach RJ., Rational development of LEA29Y (belatacept), a high-affinity variant of CTLA4-Ig with potent immunosuppressive properties. Am J Transplant, 2005. 5(3): p. 443-453.
25. Bluestone, J.A., E.W. St Clair, and L.A. Turka, CTLA4Ig: bridging the basic immunology with clinical application. Immunity, 2006. 24(3): p. 233-238.
26. Kevin Horgan, S.S., Monica Boirivant, Immunomagnetic Purification of T-Cell Subpopulations. Current Protocols in Immunology, 2009. 85: p. 7.4.1-7.4.9.
27. Karlsson AC, M.J., Younger SR, Bredt BM, Epling L, Ronquillo R, Varma A, Deeks SG, McCune JM, Nixon DF, Sinclair E., Comparison of the ELISPOT and cytokine flow cytometry assays for the enumeration of antigen-specific T cells. J Immunol Methods, 2003. 283(1-2): p. 141-153.
28. Yik Y. L. Yu, N.N., Lonnie Lybarger, Janet M. Connolly and Ted H. Hansen, Single-Chain Trimers of MHC Class I Molecules Form Stable Structures That Potently Stimulate Antigen-Specific T Cells and B Cells. The Journal of Immunology, 2002. 168(7): p. 3145-4149.
29. Sojung Kim, L.L., Curtis P. McMurtrey, William H. Hildebrand,Jon A. Weidanz,William E. Gillanders, Michael S. Diamond, and Ted H. Hansen, Single-Chain HLA-A2 MHC Trimers That Incorporate an Immundominant Peptide Elicit Protective T Cell Immunity against Lethal West Nile Virus Infection. The Journal of Immunology, 2010. 184: p. 4423-4430.
30. Samanta D, M.G., Ramagopal UA, Chaparro RJ, Nathenson SG, DiLorenzo TP, Almo SC., Structural and functional characterization of a single-chain peptide-MHC molecule that modulates both naive and activated CD8+ T cells. Proc Natl Acad Sci USA, 2011. 108(33): p. 13682-13687.
31. Whitehead, T.A., et al., Optimization of affinity, specificity and function of designed influenza inhibitors using deep sequencing. Nat Biotechnol, 2012. 30(6): p. 543-8.
32. Smith, A.M., et al., Competitive genomic screens of barcoded yeast libraries. J Vis Exp, 2011(54).
33. Lee, W., et al., Genome-wide requirements for resistance to functionally distinct DNA-damaging agents. PLoS Genet, 2005. 1(2): p. e24.
34. Pierce, S.E., et al., Genome-wide analysis of barcoded Saccharomyces cerevisiae gene-deletion mutants in pooled cultures. Nat Protoc, 2007. 2(11): p. 2958-74.
35. Satomi Nadanaka, H.Y., Fumi Kano, Masayuki Murata, and Kazutoshi Mori, Activation of Mammalian Unfolded Protein Response Is Compatible with the Quality Control System Operating in the Endoplasmic Reticulum. Molecular Biology of the Cell, 2004. 15: p. 2537-2548.
36. David N. Garboczi, D.T.H., and Don C. Wiley, HLA-A2-peptide complexes: Refolding and crystallization of molecules expressed in Eschericia Coli and complexed with single antigenic peptides. Proc Natl Acad Sci USA, 1992. 89: p. 3429-3433.
37. Andrea L Szymczak, C.J.W., Yao Wang, Kate M Vignali, Smaroula Dilioglou, Elio F Vanin and Dario A A Vignali, Correction of multi-gene deficiency in vivo using a single 'self-cleaving' 2A peptide-based retroviral vector. Nature Biotechnology, 2004. 22: p. 589-594.

## Claims

1. A (i) lentivirus-like particle or (ii) lentivirus, which lentivirus-like particle or a lentivirus is physically associated with an expression product via a mammalian cell membrane portion associated with the lentivirus-like particle or lentivirus and the mammalian transmembrane domain of the expression product, the expression product comprising in N-terminal to C-terminal order:
a 5 to 20 amino acid peptide, contiguous with
a first linker peptide sequence, contiguous with
a beta 2 microglobulin sequence, contiguous with
a second linker peptide sequence, contiguous with
a class I Major Histocompatibility Complex (MHC) heavy chain sequence, contiguous with
a third linker peptide sequence, contiguous with
a fluorescent protein or a sequence of an immunoglobulin Fc domain, contiguous with
a fourth linker peptide sequence, contiguous with
the mammalian transmembrane domain, contiguous with
a lentiviral packaging sequence comprising the GP41 envelope protein residues 706-713.

2. The lentivirus-like particle or lentivirus of claim 1, wherein the mammalian transmembrane domain is a class I MHC heavy chain transmembrane domain.

3. A plurality of isolated lentiviruses or isolated lentivirus-like particles of claim 1 or claim 2.

4. The plurality of claim 3, wherein the plurality comprises at least 100 different encoded 5 to 20 amino acid peptides.

5. An isolated suspension-adapted mammalian cell expressing an expression product of a heterologous nucleic acid transduced or transfected therein, or a membrane-bound portion of such cell expressing the expression product, which expression product comprises, in N-terminal to C-terminal order:
a 5 to 20 amino acid peptide, contiguous with
a first linker peptide sequence, contiguous with
a beta 2 microglobulin sequence, contiguous with
a second linker peptide sequence, contiguous with
a class I Major Histocompatibility Complex heavy chain sequence, contiguous with
a third linker peptide sequence, contiguous with
a fluorescent protein or a sequence of an immunoglobulin Fc domain, contiguous with
a fourth linker peptide sequence, contiguous with
a mammalian transmembrane domain, contiguous with
a lentiviral packaging sequence comprising the GP41 envelop protein residues 706-713.

6. The membrane-bound portion of the cell of claim 5, wherein the membrane-bound portion comprises the expression product.

7. The membrane-bound portion of the cell of claim 5 or 6 which is a microvesicle or an exosome.

8. The isolated suspension-adapted cell, or membrane-bound portion of such cell expressing the expression product, of any one of claims 5-7, wherein the cell expresses the expression product comprising the sequence of the immunoglobulin Fc domain.

9. The isolated suspension-adapted cell, or membrane-bound portion of such cell expressing the expression product, of any one of claims 5-7, wherein the cell expresses the expression product comprising the fluorescent protein.

10. The isolated suspension-adapted cell of any of the preceding claims, or a membrane-bound portion of such cell expressing the expression product, wherein the mammalian transmembrane domain is a Major Histocompatibility Complex heavy chain transmembrane domain.

11. A plurality of the isolated suspension-adapted cells of any one of claims 5-10, or a plurality of membrane-bound portions of such cells expressing the expression product, wherein the plurality comprises at least two different encoded 5 to 20 amino acid peptides, wherein the encoded peptide or peptides is or are presented on an extracellular surface of the cells.

12. The plurality of claim 11, wherein the plurality comprises at least 100 different encoded 5 to 20 amino acid peptides.

13. A method of identifying a T-cell epitope comprising:
contacting a T-cell with a plurality, comprising at least two cells, of isolated suspension-adapted cells according to any one of claims 5-12, or a membrane-bound portion of such cells expressing the expression product, or with lentivirus-like particles or lentiviruses according to any one of claims 1-3 associated with a membrane portion of such cells;
recovering T-cell(s) which have formed a conjugate with a suspension-adapted cell or membrane-bound portions;
recovering DNA from the suspension-adapted cell(s);
sequencing the recovered DNA; and
identifying the 5 to 20 amino acid peptide(s) encoded for in the DNA, so as to thereby identify a T-cell epitope.

14. The method of claim 13, wherein the T-cells comprise peripheral T-cells obtained from a subject, such as a human subject.

## Patentansprüche

1. (i) Lentivirus-ähnliches Teilchen oder (ii) Lentivirus, wobei das Lentivirus-ähnliche Teilchen oder ein Lentivirus physikalisch mit einem Expressionsprodukt über einen Säugetierzellmembranabschnitt verbunden ist, der mit dem Lentivirus-ähnlichen Teilchen oder dem Lentivirus und der Säugetiertransmembrandomäne des Expressionsprodukts verbunden ist, wobei das Expressionsprodukt in der Reihenfolge vom N-Terminus zum C-Terminus Folgendes umfasst:
ein Peptid aus 5 bis 20 Aminosäuren, zusammenhängend mit einer ersten Linkerpeptidsequenz, zusammenhängend mit
einer β-2-Mikroglobulin-Sequenz, zusammenhängend mit
einer zweiten Linkerpeptidsequenz, zusammenhängend mit
einer Schwerkettensequenz eines Hauptkompatibilitätskomplexes (MHC) Klasse I, zusammenhängend mit
einer dritten Linkerpeptidsequenz, zusammenhängend mit
einem fluoreszierenden Protein oder einer Sequenz einer Immunglobulin-Fc-Domäne, zusammenhängend mit
einer vierten Linkerpeptidsequenz, zusammenhängend mit
der Säugetiertransmembrandomäne, zusammenhängend mit
einer lentiviralen Verpackungssequenz, die die Reste 706-713 des GP41-Hüllenproteins umfasst.

2. Lentivirus-ähnliches Teilchen oder Lentivirus nach Anspruch 1, wobei die Säugetiertransmembrandomäne eine Transmembrandomäne einer Klasse-I-MHC-Schwerkette ist.

3. Vielzahl isolierter Lentiviren oder isolierter Lentivirus-ähnlicher Teilchen nach Anspruch 1 oder Anspruch 2.

4. Vielzahl nach Anspruch 3, wobei die Vielzahl zumindest 100 verschiedene kodierte Peptide aus 5 bis 20 Aminosäuren umfasst.

5. Isolierte Suspension-adaptierte Säugetierzelle, die ein Expressionsprodukt einer darin transduzierten oder transfizierten heterologen Nucleinsäure exprimiert, oder membrangebundener Abschnitt einer solchen Zelle, die das Expressionsprodukt exprimiert, wobei das Expressionsprodukt in der Reihenfolge vom N-Terminus zum C-Terminus Folgendes umfasst:
ein Peptid aus 5 bis 20 Aminosäuren, zusammenhängend mit einer ersten Linkerpeptidsequenz, zusammenhängend mit
einer β-2-Mikroglobulin-Sequenz, zusammenhängend mit
einer zweiten Linkerpeptidsequenz, zusammenhängend mit
einer Schwerkettensequenz eines Hauptkompatibilitätskomplexes (MHC) Klasse I, zusammenhängend mit
einer dritten Linkerpeptidsequenz, zusammenhängend mit
einem fluoreszierenden Protein oder einer Sequenz einer Immunglobulin-Fc-Domäne, zusammenhängend mit
einer vierten Linkerpeptidsequenz, zusammenhängend mit
der Säugetiertransmembrandomäne, zusammenhängend mit
einer lentiviralen Verpackungssequenz, die die Reste 706-713 des GP41-Hüllenproteins umfasst.

6. Membrangebundener Abschnitt einer Zelle nach Anspruch 5, wobei der membrangebundene Abschnitt das Expressionsprodukt umfasst.

7. Membrangebundener Abschnitt einer Zelle nach Anspruch 5 oder 6, der ein Mikrovesikel oder ein Exosom darstellt.

8. Isolierte Suspension-adaptierte Zelle oder membrangebundener Abschnitt einer solchen Zelle, die das Expressionsprodukt exprimiert, nach einem der Ansprüche 5 bis 7, wobei die Zelle das Expressionsprodukt, umfassend die Sequenz der Immunglobulin-Fc-Domäne, exprimiert.

9. Isolierte Suspension-adaptierte Zelle oder membrangebundener Abschnitt einer solchen Zelle, die das Expressionsprodukt exprimiert, nach einem der Ansprüche 5 bis 7, wobei die Zelle das Expressionsprodukt, umfassend das fluoreszierende Protein, exprimiert.

10. Isolierte Suspension-adaptierte Zelle nach einem der vorangegangenen Ansprüche oder membrangebundener Abschnitt einer solchen Zelle, die das Expressionsprodukt exprimiert, wobei die Säugetiertransmembrandomäne eine Transmembrandomäne einer Haupthistokompatibilitäts-Schwerkette ist.

11. Vielzahl isolierter Suspension-adaptierter Zellen nach einem der Ansprüche 5 bis 10 oder Vielzahl membrangebundener Abschnitte solcher Zellen, die das Expressionsprodukt exprimieren, wobei die Vielzahl zumindest zwei verschiedene kodierte Peptide aus 5 bis 20 Aminosäuren umfasst, wobei das kodierte Peptid oder die kodierten Peptide auf einer extrazellulären Oberfläche der Zellen vorhanden ist oder sind.

12. Vielzahl nach Anspruch 11, wobei die Vielzahl zumindest 100 verschiedene kodierte Peptide aus 5 bis 20 Aminosäuren umfasst.

13. Verfahren zum Identifizieren eines T-Zell-Epitops, das Folgendes umfasst:
das Inkontaktbringen einer T-Zelle mit einer Vielzahl, umfassend zumindest zwei Zellen, isolierter Suspension-adaptierter Zellen nach einem der Ansprüche 5 bis 12 oder einem membrangebundenen Abschnitt solcher Zellen, die das Expressionsprodukt exprimieren, oder mit Lentivirus-ähnlichen Teilchen oder Lentiviren nach einem der Ansprüche 1 bis 3, die mit einem Membranabschnitt solcher Zellen verbunden sind;
das Gewinnen von einer oder mehreren T-Zellen, die ein Konjugat mit einer Suspension-adaptierten Zelle oder membrangebundenen Abschnitten gebildet haben;
das Gewinnen von DNA von der oder den Suspension-adaptierten Zelle(n);
das Sequenzieren der gewonnenen DNA; und
das Identifizieren des Peptids oder der Peptide aus 5 bis 20 Aminosäuren, für die in der DNA kodiert wird, um dadurch ein T-Zell-Epitop zu identifizieren.

14. Verfahren nach Anspruch 13, wobei die T-Zellen periphere T-Zellen umfassen, die von einem Individuum wie einem menschlichen Individuum erhalten werden.

## Revendications

1. (i) Particule analogue à un lentivirus ou (ii) lentivirus, laquelle particule analogue à un lentivirus ou lequel lentivirus est physiquement associé(e) à un produit d'expression via une partie de membrane cellulaire de mammifère associée à la particule analogue à un lentivirus ou au lentivirus et au domaine transmembranaire de mammifère du produit d'expression, le produit d'expression comprenant dans l'ordre de la terminaison N à la terminaison C :
un peptide de 5 à 20 acides aminés, contigu à
une première séquence peptidique de liaison, contiguë à
une séquence de microglobuline bêta 2, contiguë à
une deuxième séquence peptidique de liaison, contiguë à
une séquence de chaîne lourde de complexe majeur d'histocompatibilité (CMH) de classe I, contiguë à
une troisième séquence peptidique de liaison, avec
une protéine fluorescente ou une séquence d'un domaine Fc d'immunoglobuline, contiguë à
une quatrième séquence peptidique de liaison, contiguë à
le domaine transmembranaire de mammifère, contigu à
une séquence d'encapsidation lentivirale comprenant les résidus de protéine d'enveloppe GP41 706-713.

2. Particule ou lentivirus analogue à un lentivirus selon la revendication 1, dans lequel le domaine transmembranaire de mammifère est un domaine transmembranaire de chaîne lourde de CMH de classe I.

3. Pluralité de lentivirus isolés ou de particules analogues à un lentivirus isolé selon la revendication 1 ou la revendication 2.

4. Pluralité selon la revendication 3, dans laquelle la pluralité comprend au moins 100 peptides codés différents de 5 à 20 acides aminés.

5. Cellule de mammifère isolée adaptée en suspension exprimant un produit d'expression d'un acide nucléique hétérologue transduit ou transfecté en son sein, ou une partie liée à une membrane d'une telle cellule exprimant le produit d'expression, lequel produit d'expression comprend, dans l'ordre de la terminaison N à la terminaison C :
un peptide de 5 à 20 acides aminés, contigu à
une première séquence peptidique de liaison, contiguë à
une séquence de microglobuline bêta 2, contiguë à
une deuxième séquence peptidique de liaison, contiguë à
une séquence de chaîne lourde de complexe majeur d'histocompatibilité (CMH) de classe I, contiguë à
une troisième séquence peptidique de liaison, avec
une protéine fluorescente ou une séquence d'un domaine Fc d'immunoglobuline, contiguë à
une quatrième séquence peptidique de liaison, contiguë à
un domaine transmembranaire de mammifère, contigu à
une séquence d'encapsidation lentivirale comprenant les résidus de protéine d'enveloppe GP41 706-713.

6. Partie liée à une membrane de la cellule selon la revendication 5, dans laquelle la partie liée à une membrane comprend le produit d'expression.

7. Partie liée à une membrane de la cellule selon la revendication 5 ou 6 qui est une microvésicule ou un exosome.

8. Cellule isolée adaptée en suspension, ou partie liée à une membrane d'une telle cellule exprimant le produit d'expression, selon l'une quelconque des revendications 5 à 7, dans laquelle la cellule exprime le produit d'expression comprenant la séquence du domaine Fc d'immunoglobuline.

9. Cellule isolée adaptée en suspension, ou partie liée à une membrane d'une telle cellule exprimant le produit d'expression, selon l'une quelconque des revendications 5 à 7, dans laquelle la cellule exprime le produit d'expression comprenant la protéine fluorescente.

10. Cellule isolée adaptée en suspension selon l'une quelconque des revendications précédentes, ou partie liée à une membrane d'une telle cellule exprimant le produit d'expression, dans laquelle le domaine transmembranaire de mammifère est un domaine transmembranaire de chaîne lourde du complexe majeur d'histocompatibilité.

11. Pluralité de cellules isolées adaptées en suspension selon l'une quelconque des revendications 5 à 10, ou pluralité de parties liées à une membrane de telles cellules exprimant le produit d'expression, dans laquelle la pluralité comprend au moins deux peptides codés différents de 5 à 20 acides aminés, dans laquelle le peptide codé ou les peptides codés est ou sont présentés sur une surface extracellulaire des cellules.

12. Pluralité selon la revendication 11, dans laquelle la pluralité comprend au moins 100 peptides codés différents de 5 à 20 acides aminés.

13. Procédé d'identification d'un épitope de cellule T comprenant les étapes consistant à :
mettre en contact une cellule T avec une pluralité, comprenant au moins deux cellules, de cellules isolées adaptées en suspension selon l'une quelconque des revendications 5 à 12, ou une partie liée à une membrane de telles cellules exprimant le produit d'expression, ou avec des particules analogues à un lentivirus ou des lentivirus selon l'une quelconque des revendications 1 à 3 associé(e)s à une partie membranaire de telles cellules ;
récupérer une ou des cellules T qui ont formé un conjugué avec une cellule adaptée en suspension ou des parties liées à une membrane ;
récupérer de l'ADN à partir de la ou des cellules adaptées en suspension ;
séquencer l'ADN récupéré ; et
identifier le ou les peptides de 5 à 20 acides aminés codés dans l'ADN, de manière à identifier ainsi un épitope de cellule T.

14. Procédé selon la revendication 13, dans lequel les cellules T comprennent des cellules T périphériques obtenues auprès d'un sujet, comme un sujet humain.
